# EUROPEAN PATENT APPLICATION

(11) **EP 3 748 018 A1**
(43) Date of publication of application: **09.12.2020**
(21) Application number: 19744087.8
(22) Date of filing: 25.01.2019
(51) Int. Cl.: C12Q 1/689

(54) **METHOD FOR DIAGNOSING DEPRESSION VIA BACTERIAL METAGENOMIC ANALYSIS**

(30) Priority: 29.01.2018 KR 20180010409
(71) Applicant: MD Healthcare Inc., Seoul 03923 (KR)
(72) Inventor: KIM, Yoon-Keun, Paju-si, Gyeonggi-do 10908 (KR)
(74) Representative: Dehns
(86) International application number: PCT/KR2019/001121
(87) International publication number: WO 2019/147080

(57) **Abstract**

The present invention relates to a method of diagnosing depression through bacterial metagenomic analysis, and more particularly to a method of diagnosing depression by analyzing an increase or decrease in content of specific bacteria-derived extracellular vesicles through bacterial metagenomic analysis using subject-derived samples, and the like. Extracellular vesicles secreted from microorganisms such as bacteria and archaea, present in the environment, are absorbed into the body and thus can directly affect the occurrence of inflammation, and depression characterized by an inflammatory response is difficult to diagnose early before symptoms appear so that effective treatment is difficult. Therefore, by previous diagnosis of the risk for the onset of depression through metagenomic analysis of bacteria-derived extracellular vesicles using human-derived samples according to the present invention, the risk group of depression can be diagnosed and predicted early to delay or prevent the onset of depression through proper management, and early diagnosis can be performed even after the onset, thereby reducing the incidence of depression and increasing the therapeutic effect.

<110>    MD Healthcare Inc.

<120>    Method for diagnosis of depression using analysis of bacteria
         metagenome

<130>    MPCT18-088

<150>    KR 10-2018-0010409
<151>    2018-01-29

<160>    2

<170>    KoPatentIn 3.0

<210>    1
<211>    50
<212>    DNA
<213>    Artificial Sequence

<220>
<223>    16S_V3_F


<400>    1
tcgtcggcag cgtcagatgt gtataagaga cagcctacgg gnggcwgcag                50


<210>    2
<211>    55
<212>    DNA
<213>    Artificial Sequence

<220>
<223>    16S_V4_R


<400>    2
gtctcgtggg ctcggagatg tgtataagag acaggactac hvgggtatct aatcc          55

## Description

### [Technical Field]

The present invention relates to a method for diagnosing depression through a bacterial metagenomic analysis and, more specifically, to a method of diagnosing depression, and the like by performing a bacterial metagenomic analysis using subject-derived samples to analyze an increase or decrease in the content of specific bacteria-derived extracellular vesicles.

### [Background Art]

Depression is a common disorder that causes a "negative feeling" due to a change in brain function controlling feelings. Globally, more than 100 million people suffer from depression. The nature of depression is derived from physiological and anatomical problems, and negative feelings are the result of a change in the structure of the human body. Depression may be classified as mild and severe according to the degree thereof. Mild depression is depression that is diagnosed but there is no problem in social activity such as occupational activity, etc. Severe depression is also called "depressive disorder" when a variety of depressive symptoms persist in the mental health system, and "major depressive disorder" when a variety of specific symptoms persist for a considerable amount of time.

Scientists are trying to determine the cause of depression, but the exact cause of the outbreak has not been revealed. Serotonin and melatonin are representative substsancess that are recognized as the cause of depression, and several hormones related to nerves, such as dopamine and norepinephrine also affect depression. As assumed from the terms such as pregnancy depression, postpartum depression, housewife depression and seasonal depression, the onset of depression is affected internally and externally. Serotonin is a neurometabolite found in the cerebrospinal fluid, and circulates throughout the brain and functions as a neurotransmitter. Serotonin is closely related to emotional expression, and when this substance is insufficient, emotions become unstable, resulting in increasing anxiety and worries and impulsive tendencies. In the 1970s, scientists revealed that serotonin deficiency was closely related to depression. Among drugs currently used as depression therapeutic agents, there are many drugs that prevent reabsorption of serotonin so that it stays in the brain for longer. Depression is generally twice as common in women as in men. Women generally have higher serotonin levels than men, but since they are sensitive to a slight change in serotonin level, women are more likely to develop depression. It is interpreted that the reason why women respond more sensitively than men is that a serotonin level is changed by stimulation of the brain due to the imbalance of female hormones such as estrogen and progesterone before and after the menstrual cycle.

Meanwhile, it is known that the number of microorganisms symbiotically living in the human body is 100 trillion which is 10 times the number of human cells, and the number of genes of microorganisms exceeds 100 times the number of human genes. A microbiota or microbiome is a microbial community that includes bacteria, archaea, and eukaryotes present in a given habitat. The intestinal microbiota is known to play a vital role in human's physiological phenomena and significantly affect human health and diseases through interactions with human cells. Bacteria coexisting in human bodies secrete nanometer-sized vesicles to exchange information about genes, proteins, low molecular weight compound, and the like with other cells. The mucous membranes form a physical barrier membrane that does not allow particles with the size of 200 nm or more to pass therethrough, and thus bacteria symbiotically living in the mucous membranes are unable to pass therethrough, but bacteria-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment (Korean Patent Publication No. 2011-0073049). Recently, the bacterial composition of human microbiota has been listed using a method based on 16s ribosomal RNA (16s rRNA) base sequences, and 16s rDNA base sequences, which are genes of 16s ribosomal RNA, are analyzed using a next generation sequencing (NGS) platform. However, as for the occurrence of depression, there is no report about a method of identifying, from a human-derived material such as urine, a causative factor of depression by analysis of metagenomes present in bacteria-derived vesicles and of diagnosing depression.

### [Disclosure]

### [Technical Problem]

The present inventors extracted genes from bacteria-derived extracellular vesicles present in urine as subject-derived samples and performed a metagenomic analysis in this regard in order to diagnose the causal factors and risk of depression in advance, and as a result, identified bacteria-derived extracellular vesicles which may act as a causal factor of depression, thereby completing the present invention based on this.

Therefore, an object of the present invention is to provide a method of providing information for diagnosing depression through the metagenomic analysis of bacteria-derived extracellular vesicles.

However, the technical goals of the present invention are not limited to the aforementioned goals, and other unmentioned technical goals will be clearly understood by those of ordinary skill in the art from the following description.

### [Technical Solution]

To achieve the above-described object of the present invention, there is provided a method of providing information for depression diagnosis, comprising the following processes:
(a) extracting DNAs from extracellular vesicles isolated from subject samples;
(b) performing polymerase chain reaction (PCR) on the extracted DNA using a pair of primers comprising SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

The present invention also provides a method of diagnosing depression, comprising the following processes:
(a) extracting DNAs from extracellular vesicles isolated from subject samples;
(b) performing polymerase chain reaction (PCR) on the extracted DNA using a pair of primers comprising SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

The present invention also provides a method of predicting a risk for depression, comprising the following processes:
(a) extracting DNAs from extracellular vesicles isolated from subject samples;
(b) performing polymerase chain reaction (PCR) on the extracted DNA using a pair of primers comprising SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

In another embodiment of the present invention, in process (c), the depression may be diagnosed by comparing an increase or decrease in content of extracellular vesicles derived from one or more bacteria selected from the group consisting of the phylum *Verrucomicrobia,* the phylum *Bacteroidetes,* the phylum *Actinobacteria,* the phylum *Cyanobacteria,* the phylum *Thermi,* the phylum *Fusobacteria,* the phylum *Acidobacteria,* the phylum *Chloroflexi,* and the phylum *Armatimonadetes.*

In another embodiment of the present invention, in process (c), the depression may be diagnosed by comparing an increase or decrease in content of extracellular vesicles derived from one or more bacteria selected from the group consisting of the class *Erysipelotrichi,* the class *Verrucomicrobiae,* the class *Bacteroidia,* the class *Clostridia,* the class *Chloroplast,* the class *Actinobacteria,* the class *Alphaproteobacteria,* the class *Deltaproteobacteria,* the class *Saprospirae,* the class *Flavobacteriia,* the class *Cytophagia,* the class *Deinococci,* the class *Sphingobacteriia,* the class *Fusobacteriia,* the class *Fimbriimonadia,* the class TM7-1, the class *Pedosphaerae,* the class *Oscillatoriophycideae,* the class *Anaerolineae,* the class *Acidobacteria*-6, the class *Solibacteres,* the class *Nostocophycideae,* and the class iii1-8.

In another embodiment of the present invention, in process (c), the depression may be diagnosed by comparing an increase or decrease in content of extracellular vesicles derived from one or more bacteria selected from the group consisting of the order *Turicibacterales,* the order *Erysipelotrichales,* the order *Verrucomicrobiales,* the order RF39, the order *Bacteroidales,* the order *Enterobacteriales,* the order *Clostridiales,* the order *Pasteurellales,* the order *Sphingomonadales,* the order *Bacillales,* the order *Xanthomonadales,* the order *Streptophyta,* the order *Rhodobacterales,* the order *Caulobacterales,* the order *Actinomycetales,* the order *Saprospirales,* the order *Rhizobiales,* the order *Flavobacteriales,* the order *Cytophagales,* the order *Myxococcales,* the order *Rickettsiales,* the order *Thermales,* the order *Rhodospirillales,* the order *Deinococcales,* the order *Sphingobacteriales,* the order *Fusobacteriales,* the order *Fimbriimonadales,* the order BD7-3, the order *Pedosphaerales,* the order *Aeromonadales,* the order *Chroococcales,* the order 1025, the order *Bdellovibrionales,* the order iii1-15, the order *Solibacterales,* the order PYR10d3, and the order DS-18.

In another embodiment of the present invention, in process (c), the depression may be diagnosed by comparing an increase or decrease in content of extracellular vesicles derived from one or more bacteria selected from the group consisting of the family *Turicibacteraceae,* the family *Erysipelotrichaceae,* the family *Verrucomicrobiaceae,* the family *Rikenellaceae,* the family *Bacteroidaceae,* the family *Odoribacteraceae,* the family S24-7, the family *Clostridiaceae,* the family *Ruminococcaceae*, the family *Enterococcaceae,* the family *Paraprevotellaceae,* the family *Enterobacteriaceae,* the family *Leuconostocaceae,* the family *Porphyromonadaceae*, the family *Prevotellaceae,* the family *Pasteurellaceae,* the family *Actinomycetaceae,* the family *Burkholderiaceae,* the family *Xanthomonadaceae*, the family *Intrasporangiaceae,* the family *Staphylococcaceae,* the family *Sphingomonadaceae,* the family *Propionibacteriaceae,* the family *Nocardiaceae,* the family *Lactobacillaceae,* the family *Methylobacteriaceae,* the family *Rhodobacteraceae,* the family *Tissierellaceae,* the family *Acetobacteraceae*, the family *Caulobacteraceae,* the family *Nocardioidaceae*, the family *Hyphomicrobiaceae,* the family *Chitinophagaceae,* the family *Chromatiaceae,* the family *Flavobacteriaceae,* the family *Micrococcaceae,* the family *Sphingobacteriaceae,* the family *Corynebacteriaceae,* the family *Cytophagaceae,* the family *Weeksellaceae*, the family *Sinobacteraceae,* the family *Thermaceae,* the family *Deinococcaceae,* the family *Rhizobiaceae,* the family *Mycobacteriaceae,* the family *Comamonadaceae,* the family *Bacillaceae,* the family *Dermabacteraceae,* the family *Erythrobacteraceae,* the family *Geodermatophilaceae,* the family *Fimbriimonadaceae*, the family *Bartonellaceae,* the family *Leptotrichiaceae,* the family *Dermacoccaceae,* the family *Rhodospirillaceae,* the family *Aeromonadaceae*, the family Rs-045, the family *Xenococcaceae,* the family *Sporichthyaceae,* the family *Bdellovibrionaceae,* and the family *Solibacteraceae.*

In another embodiment of the present invention, in process (c), the depression may be diagnosed by comparing an increase or decrease in content of extracellular vesicles derived from one or more bacteria selected from the group consisting of the genus *Butyricimonas,* the genus *Turicibacter,* the genus SMB53, the genus *Eubacterium,* the genus *Akkermansia*, the genus *Proteus,* the genus *Ruminococcus*, the genus *Parabacteroides,* the genus *Roseburia,* the genus *Bacteroides,* the genus *Phascolarctobacterium,* the genus *Sporosarcina,* the genus *Weissella,* the genus *Faecalibacterium,* the genus *Collinsella,* the genus *Oscillospira,* the genus *Prevotella,* the genus *Sphingomonas,* the genus *Finegoldia*, the genus *Chryseobacterium,* the genus *Lautropia,* the genus *Methylobacterium,* the genus *Propionibacterium,* the genus *Rhodococcus,* the genus *Flavobacterium,* the genus *Staphylococcus,* the genus *Lactobacillus,* the genus *Porphyromonas,* the genus *Peptoniphilus,* the genus *Adlercreutzia,* the genus *Erwinia,* the genus *Citrobacter,* the genus *Microbispora,* the genus *Corynebacterium,* the genus *Leptotrichia,* the genus *Pedobacter,* the genus *Enhydrobacter,* the genus *Cloacibacterium*, the genus *Paracoccus,* the genus *Kaistobacter,* the genus *Thermus*, the genus *Actinobacillus,* the genus *Deinococcus,* the genus *Anaerococcus,* the genus *Hymenobacter,* the genus *Achromobacter,* the genus *Mycobacterium,* the genus *Agrobacterium,* the genus *Brachybacterium*, the genus *Capnocytophaga,* the genus *Bacillus,* the genus *Arthrobacter,* the genus *Megasphaera*, the genus *Fimbriimonas,* the genus *Peptostreptococcus,* the genus *Modestobacter,* the genus *Tepidimonas*, the genus *Dermacoccus*, the genus *Janthinobacterium,* the genus *Wautersiella*, the genus *Novosphingobium,* the genus *Nesterenkonia,* the genus *Sneathia*, the genus WAL_1855D, the genus *Skermanella,* the genus *Shuttleworthia,* the genus *Roseateles,* the genus *Bdellovibrio,* the genus *Actinobaculum,* the genus *Candidatus Solibacter,* and the genus *Chroococcidiopsis.*

In another embodiment of the present invention, in process (c), in comparison with the normal individual-derived sample, it is possible to diagnose an increase in the content of the following as depression:
extracellular vesicles derived from one or more bacteria selected from the group consisting of the phylum *Actinobacteria,* the phylum *Cyanobacteria,* the phylum *Thermi,* the phylum *Fusobacteria,* the phylum *Acidobacteria,* the phylum *Chloroflexi,* and the phylum *Armatimonadetes,*
extracellular vesicles derived from one or more bacteria selected from the group consisting of the class *Chloroplast,* the class *Actinobacteria,* the class *Alphaproteobacteria,* the class *Deltaproteobacteria,* the class *Saprospirae,* the class *Flavobacteriia,* the class *Cytophagia,* the class *Deinococci,* the class *Sphingobacteriia,* the class *Fusobacteriia,* the class *Fimbriimonadia,* the class TM7-1, the class *Pedosphaerae*, the class *Oscillatoriophycideae,* the class *Anaerolineae,* the class *Acidobacteria*-6, the class *Solibacteres,* the class *Nostocophycideae,* and the class iii1-8,
extracellular vesicles derived from one or more bacteria selected from the group consisting of the order *Pasteurellales*, the order *Sphingomonadales,* the order *Bacillales,* the order *Xanthomonadales,* the order *Streptophyta*, the order *Rhodobacterales,* the order *Caulobacterales,* the order *Actinomycetales,* the order *Saprospirales,* the order *Rhizobiales,* the order *Flavobacteriales,* the order *Cytophagales,* the order *Myxococcales,* the order *Rickettsiales,* the order *Thermales,* the order *Rhodospirillales,* the order *Deinococcales,* the order *Sphingobacteriales,* the order *Fusobacteriales,* the order *Fimbriimonadales*, the order BD7-3, the order *Pedosphaerales,* the order *Aeromonadales,* the order *Chroococcales,* the order 1025, the order *Bdellovibrionales,* the order iii1-15, the order *Solibacterales*, the order PYR10d3, and the order DS-18,
extracellular vesicles derived from one or more bacteria selected from the group consisting of the family *Pasteurellaceae*, the family *Actinomycetaceae,* the family *Burkholderiaceae,* the family *Xanthomonadaceae,* the family *Intrasporangiaceae*, the family *Staphylococcaceae,* the family *Sphingomonadaceae,* the family *Propionibacteriaceae,* the family *Nocardiaceae*, the family *Lactobacillaceae,* the family *Methylobacteriaceae,* the family *Rhodobacteraceae,* the family *Tissierellaceae*, the family *Acetobacteraceae,* the family *Caulobacteraceae,* the family *Nocardioidaceae*, the family *Hyphomicrobiaceae,* the family *Chitinophagaceae,* the family *Chromatiaceae,* the family *Flavobacteriaceae,* the family *Micrococcaceae,* the family *Sphingobacteriaceae,* the family *Corynebacteriaceae,* the family *Cytophagaceae,* the family *Weeksellaceae*, the family *Sinobacteraceae,* the family *Thermaceae,* the family *Deinococcaceae,* the family *Rhizobiaceae,* the family *Mycobacteriaceae,* the family *Comamonadaceae,* the family *Bacillaceae,* the family *Dermabacteraceae,* the family *Erythrobacteraceae,* the family *Geodermatophilaceae,* the family *Fimbriimonadaceae*, the family *Bartonellaceae,* the family *Leptotrichiaceae,* the family *Dermacoccaceae,* the family *Rhodospirillaceae,* the family *Aeromonadaceae*, the family Rs-045, the family *Xenococcaceae,* the family *Sporichthyaceae,* the family *Bdellovibrionaceae,* and the family *Solibacteraceae,* or
extracellular vesicles derived from one or more bacteria selected from the group consisting of the genus *Sphingomonas,* the genus *Finegoldia*, the genus *Chryseobacterium,* the genus *Lautropia,* the genus *Methylobacterium,* the genus *Propionibacterium,* the genus *Rhodococcus,* the genus *Flavobacterium,* the genus *Staphylococcus,* the genus *Lactobacillus,* the genus *Porphyromonas,* the genus *Peptoniphilus,* the genus *Adlercreutzia,* the genus *Erwinia,* the genus *Citrobacter,* the genus *Microbispora,* the genus *Corynebacterium,* the genus *Leptotrichia,* the genus *Pedobacter,* the genus *Enhydrobacter,* the genus *Cloacibacterium,* the genus *Paracoccus,* the genus *Kaistobacter,* the genus *Thermus*, the genus *Actinobacillus,* the genus *Deinococcus,* the genus *Anaerococcus,* the genus *Hymenobacter,* the genus *Achromobacter,* the genus *Mycobacterium,* the genus *Agrobacterium,* the genus *Brachybacterium,* the genus *Capnocytophaga*, the genus *Bacillus,* the genus *Arthrobacter,* the genus *Megasphaera*, the genus *Fimbriimonas,* the genus *Peptostreptococcus,* the genus *Modestobacter,* the genus *Tepidimonas*, the genus *Dermacoccus*, the genus *Janthinobacterium*, the genus *Wautersiella*, the genus *Novosphingobium,* the genus *Nesterenkonia,* the genus *Sneathia*, the genus WAL_1855D, the genus *Skermanella,* the genus *Shuttleworthia,* the genus *Roseateles,* the genus *Bdellovibrio,* the genus *Actinobaculum,* the genus *Candidatus Solibacter,* and the genus *Chroococcidiopsis.*

In another embodiment of the present invention, in process (c), in comparison with the normal individual-derived sample, it is possible to diagnose a decrease in the content of the following as depression:
extracellular vesicles derived from one or more bacteria selected from the group consisting of the phylum *Verrucomicrobia* and the phylum *Bacteroidetes,*
extracellular vesicles derived from one or more bacteria selected from the group consisting of the class *Erysipelotrichi,* the class *Verrucomicrobiae,* the class *Bacteroidia,* and the class *Clostridia,*
extracellular vesicles derived from one or more bacteria selected from the group consisting of the order *Turicibacterales,* the order *Erysipelotrichales,* the order *Verrucomicrobiales*, the order RF39, the order *Bacteroidales,* the order *Enterobacteriales,* and the order *Clostridiales*,
extracellular vesicles derived from one or more bacteria selected from the group consisting of the family *Turicibacteraceae,* the family *Erysipelotrichaceae,* the family *Verrucomicrobiaceae,* the family *Rikenellaceae,* the family *Bacteroidaceae,* the family *Odoribacteraceae,* the family S24-7, the family *Clostridiaceae,* the family *Ruminococcaceae*, the family *Enterococcaceae,* the family *Paraprevotellaceae,* the family *Enterobacteriaceae,* the family *Leuconostocaceae,* the family *Porphyromonadaceae*, and the family *Prevotellaceae,* or
extracellular vesicles derived from one or more bacteria selected from the group consisting of the genus *Butyricimonas,* the genus *Turicibacter,* the genus SMB53, the genus *Eubacterium,* the genus *Akkermansia*, the genus *Proteus,* the genus *Ruminococcus*, the genus *Parabacteroides,* the genus *Roseburia,* the genus *Bacteroides,* the genus *Phascolarctobacterium,* the genus *Sporosarcina,* the genus *Weissella,* the genus *Faecalibacterium,* the genus *Collinsella,* the genus *Oscillospira,* and the genus *Prevotella.*

In another embodiment of the present invention, in process (c), in comparison with the normal individual-derived sample, depression may be diagnosed when contents of vesicles derived from bacteria belonging to the genus *Corynebacterium* and the genus *Enhydrobacter* are increased, and contents of vesicles derived from bacteria belonging to the genus *Bacteroides* and the genus *Akkermansia* are decreased.

In another embodiment of the present invention, in process (c), in comparison with the normal individual-derived sample, depression may be diagnosed when contents of vesicles derived from bacteria belonging to the genus *Corynebacterium* and the genus *Enhydrobacter* are increased, and contents of vesicles derived from bacteria belonging to the genus *Bacteroides* and the genus *Akkermansia* are decreased; and
contents of extracellular vesicles derived from one or more bacteria selected from the group consisting of the phylum *Actinobacteria,* the phylum *Cyanobacteria,* the phylum *Thermi,* the phylum *Fusobacteria,* the phylum *Acidobacteria,* the phylum *Chloroflexi* and the phylum *Armatimonadetes,*
extracellular vesicles derived from one or more bacteria selected from the group consisting of the class *Chloroplast,* the class *Actinobacteria,* the class *Alphaproteobacteria,* the class *Deltaproteobacteria,* the class *Saprospirae,* the class *Flavobacteriia,* the class *Cytophagia,* the class *Deinococci,* the class *Sphingobacteriia,* the class *Fusobacteriia,* the class *Fimbriimonadia,* the class *Pedosphaerae,* the class *Oscillatoriophycideae,* the class *Anaerolineae,* the class *Acidobacteria*-6, the class *Solibacteres* and the class *Nostocophycideae,*
extracellular vesicles derived from one or more bacteria selected from the group consisting of the order *Pasteurellales,* the order *Sphingomonadales,* the order *Bacillales,* the order *Xanthomonadales,* the order *Streptophyta*, the order *Rhodobacterales,* the order *Caulobacterales,* the order *Actinomycetales,* the order *Saprospirales*, the order *Rhizobiales,* the order *Flavobacteriales,* the order *Cytophagales,* the order *Myxococcales,* the order *Rickettsiales,* the order *Thermales,* the order *Rhodospirillales,* the order *Deinococcales,* the order *Sphingobacteriales,* the order *Fusobacteriales,* the order *Fimbriimonadales*, the order *Pedosphaerales,* the order *Aeromonadales,* the order *Chroococcales,* the order *Bdellovibrionales* and the order *Solibacterales,*
extracellular vesicles derived from one or more bacteria selected from the group consisting of the family *Pasteurellaceae,* the family *Actinomycetaceae,* the family *Burkholderiaceae,* the family *Xanthomonadaceae,* the family *Intrasporangiaceae*, the family *Staphylococcaceae,* the family *Sphingomonadaceae,* the family *Propionibacteriaceae,* the family *Nocardiaceae,* the family *Lactobacillaceae,* the family *Methylobacteriaceae,* the family *Rhodobacteraceae,* the family *Tissierellaceae*, the family *Acetobacteraceae,* the family *Caulobacteraceae,* the family *Nocardioidaceae*, the family *Hyphomicrobiaceae*, the family *Chitinophagaceae,* the family *Chromatiaceae,* the family *Flavobacteriaceae,* the family *Micrococcaceae,* the family *Sphingobacteriaceae,* the family *Corynebacteriaceae,* the family *Cytophagaceae,* the family *Weeksellaceae*, the family *Sinobacteraceae,* the family *Thermaceae,* the family *Deinococcaceae,* the family *Rhizobiaceae,* the family *Mycobacteriaceae,* the family *Comamonadaceae,* the family *Bacillaceae,* the family *Dermabacteraceae,* the family *Erythrobacteraceae,* the family *Geodermatophilaceae,* the family *Fimbriimonadaceae*, the family *Bartonellaceae,* the family *Leptotrichiaceae,* the family *Dermacoccaceae,* the family *Rhodospirillaceae,* the family *Aeromonadaceae*, the family *Xenococcaceae,* the family *Sporichthyaceae,* the family *Bdellovibrionaceae* and the family *Solibacteraceae,* or
extracellular vesicles derived from one or more bacteria selected from the group consisting of the genus *Sphingomonas,* the genus *Finegoldia*, the genus *Chryseobacterium,* the genus *Lautropia,* the genus *Methylobacterium,* the genus *Propionibacterium,* the genus *Rhodococcus,* the genus *Flavobacterium,* the genus *Staphylococcus,* the genus *Lactobacillus,* the genus *Porphyromonas,* the genus *Peptoniphilus,* the genus *Adlercreutzia,* the genus *Erwinia,* the genus *Citrobacter,* the genus *Microbispora,* the genus *Leptotrichia,* the genus *Pedobacter,* the genus *Cloacibacterium*, the genus *Paracoccus,* the genus *Kaistobacter,* the genus *Thermus,* the genus *Actinobacillus*, the genus *Deinococcus,* the genus *Anaerococcus,* the genus *Hymenobacter,* the genus *Achromobacter,* the genus *Mycobacterium,* the genus *Agrobacterium,* the genus *Brachybacterium,* the genus *Capnocytophaga,* the genus *Bacillus,* the genus *Arthrobacter,* the genus *Megasphaera,* the genus *Fimbriimonas,* the genus *Peptostreptococcus,* the genus *Modestobacter,* the genus *Tepidimonas,* the genus *Dermacoccus*, the genus *Janthinobacterium,* the genus *Wautersiella,* the genus *Novosphingobium,* the genus *Nesterenkonia,* the genus *Sneathia,* the genus *Skermanella,* the genus *Shuttleworthia,* the genus *Roseateles,* the genus *Bdellovibrio,* the genus *Actinobaculum,* the genus *Candidatus Solibacter* and the genus *Chroococcidiopsis* are increased.

In another embodiment of the present invention, in process (c), in comparison with the normal individual-derived sample, depression may be diagnosed when contents of vesicles derived from bacteria belonging to the genus *Corynebacterium* and the genus *Enhydrobacter* are increased, and contents of vesicles derived from bacteria belonging to the genus *Bacteroides* and the genus *Akkermansia* are decreased; and
contents of extracellular vesicles derived from one or more bacteria selected from the group consisting of the phylum *Verrucomicrobia* and the phylum *Bacteroidetes,*
extracellular vesicles derived from one or more bacteria selected from the group consisting of the class *Erysipelotrichi,* the class *Verrucomicrobiae,* the class *Bacteroidia,* and the class *Clostridia,*
extracellular vesicles derived from one or more bacteria selected from the group consisting of the order *Turicibacterales,* the order *Erysipelotrichales,* the order *Verrucomicrobiales*, the order *Bacteroidales,* the order *Enterobacteriales,* and the order *Clostridiales,*
extracellular vesicles derived from one or more bacteria selected from the group consisting of the family *Turicibacteraceae,* the family *Erysipelotrichaceae,* the family *Verrucomicrobiaceae,* the family *Rikenellaceae,* the family *Bacteroidaceae,* the family *Odoribacteraceae,* the family *Clostridiaceae,* the family *Ruminococcaceae*, the family *Enterococcaceae,* the family *Paraprevotellaceae,* the family *Enterobacteriaceae,* the family *Leuconostocaceae,* the family *Porphyromonadaceae*, and the family *Prevotellaceae,* or
extracellular vesicles derived from one or more bacteria selected from the group consisting of the genus *Butyricimonas,* the genus *Turicibacter,* the genus *Eubacterium,* the genus *Proteus,* the genus *Ruminococcus*, the genus *Parabacteroides,* the genus *Roseburia,* the genus *Phascolarctobacterium,* the genus *Sporosarcina,* the genus *Weissella,* the genus *Faecalibacterium,* the genus *Collinsella,* the genus *Oscillospira,* and the genus *Prevotella* are decreased.

In one embodiment of the present invention, the subject sample may be urine.

### [Advantageous Effects]

Extracellular vesicles secreted from microorganisms such as bacteria and archaea, present in the environment, are absorbed into the body and thus can directly affect the occurrence of inflammation, and depression characterized by an inflammatory response is difficult to diagnose early before symptoms appear so that effective treatment is difficult. Therefore, by previous diagnosis of the risk for the onset of depression through metagenomic analysis of bacteria-derived extracellular vesicles using human-derived samples according to the present invention, the risk group of depression can be diagnosed and predicted early to delay or prevent the onset of depression through proper management, and early diagnosis can be performed even after the onset, thereby reducing the incidence of depression and increasing the therapeutic effect. In addition, the progression of cancer can be improved or the recurrence of cancer may be prevented by avoiding the exposure of causative factors through metagenomic analysis for patients diagnosed with depression.

### [Description of Drawings]

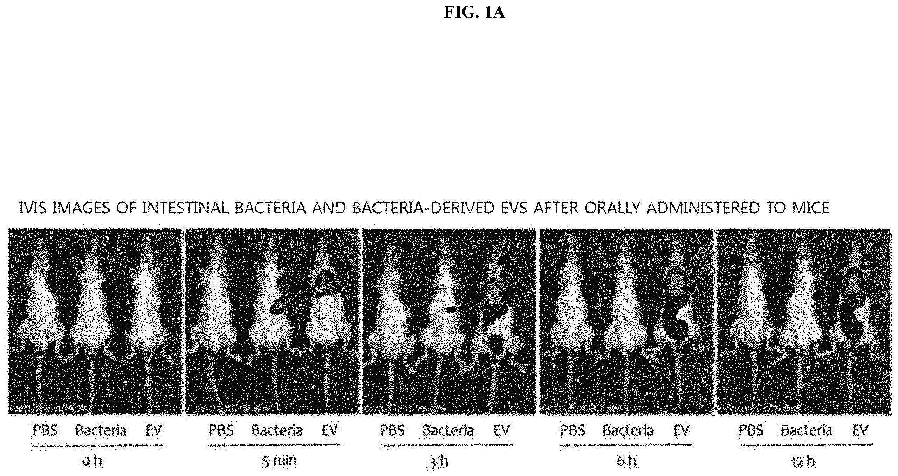

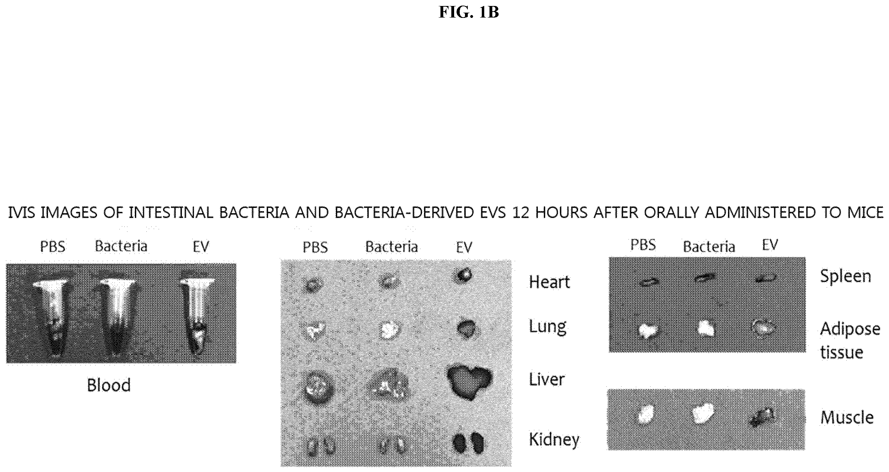

FIG. 1A illustrates images showing the distribution pattern of bacteria and extracellular vesicles over time after intestinal bacteria and bacteria-derived extracellular vesicles (EVs) were orally administered to mice, and FIG. 1B illustrates images showing the distribution pattern of bacteria and EVs after being orally administered to mice and, at 12 hours, urine and various organs were extracted.

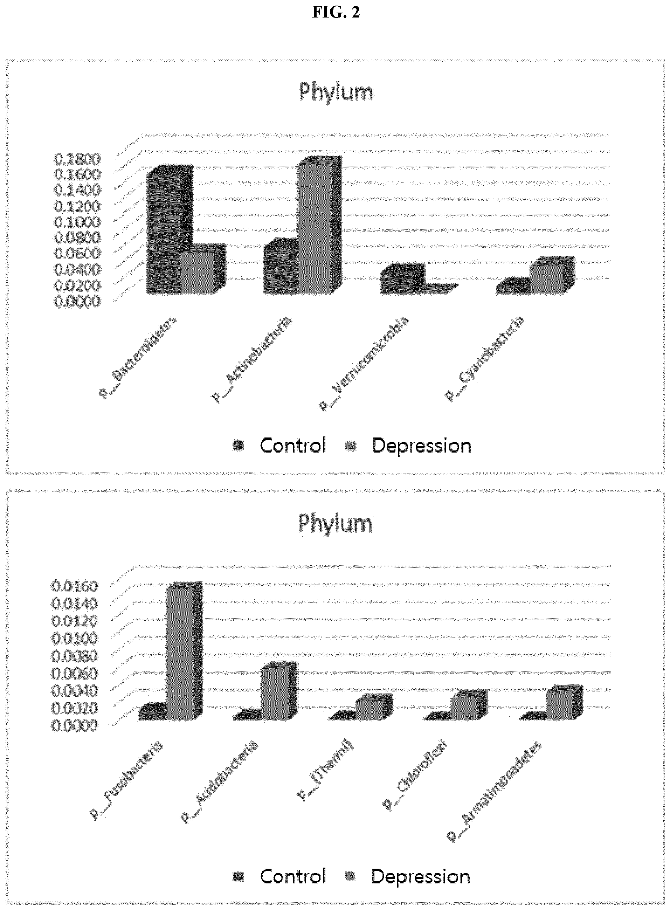

FIG. 2 is a result showing the distribution of bacteria-derived extracellular vesicles (EVs), which is significant in diagnostic performance at the phylum level by isolating bacteria-derived vesicles from urine of a patient with depression and a normal individual, and then performing a metagenomic analysis.

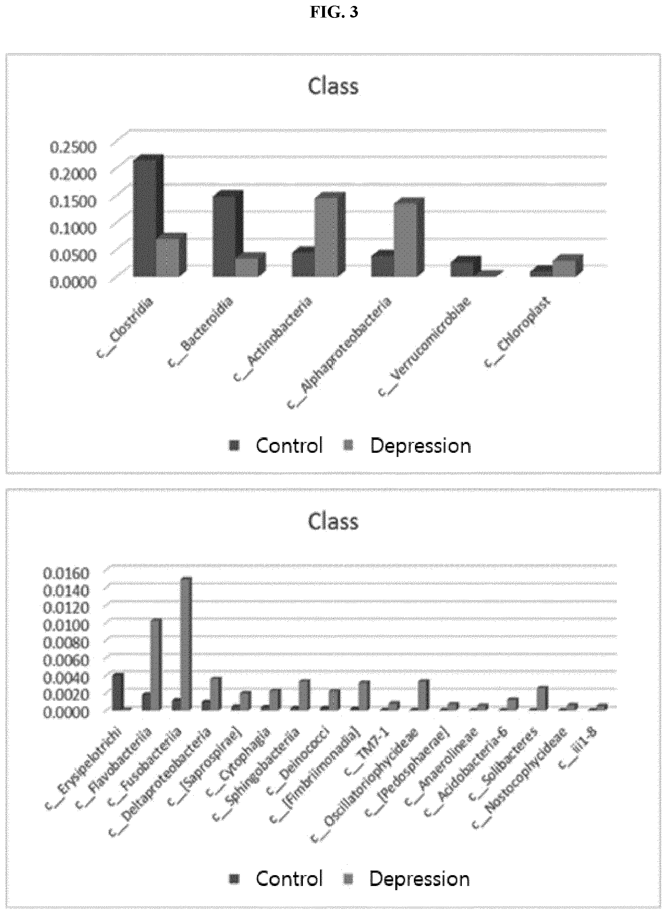

FIG. 3 is a result showing the distribution of bacteria-derived extracellular vesicles (EVs), which is significant in diagnostic performance at the class level by isolating bacteria-derived vesicles from urine of a patient with depression and a normal individual, and then performing a metagenomic analysis.

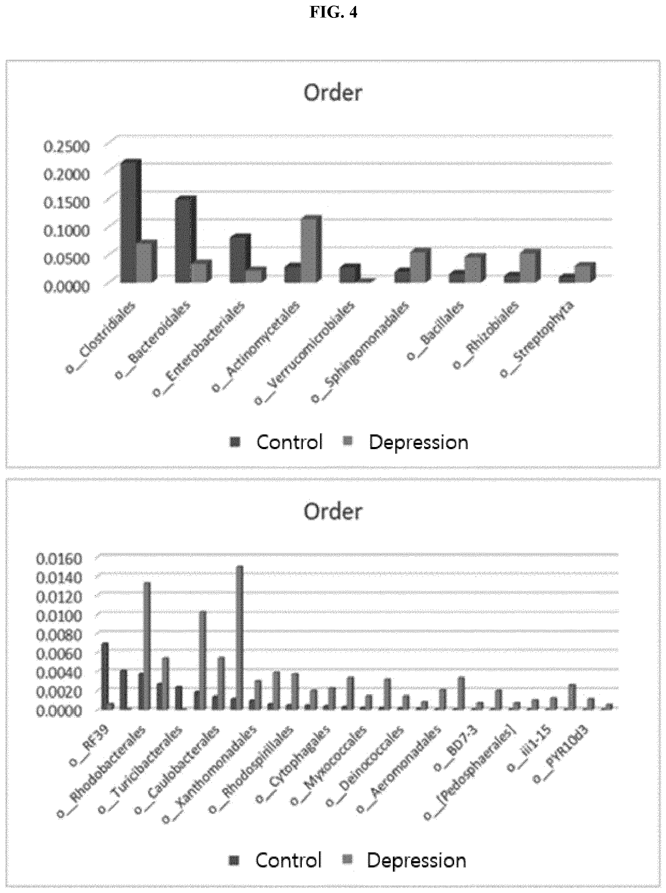

FIG. 4 is a result showing the distribution of bacteria-derived extracellular vesicles (EVs), which is significant in diagnostic performance at the order level by isolating bacteria-derived vesicles from urine of a patient with depression and a normal individual, and then performing a metagenomic analysis.

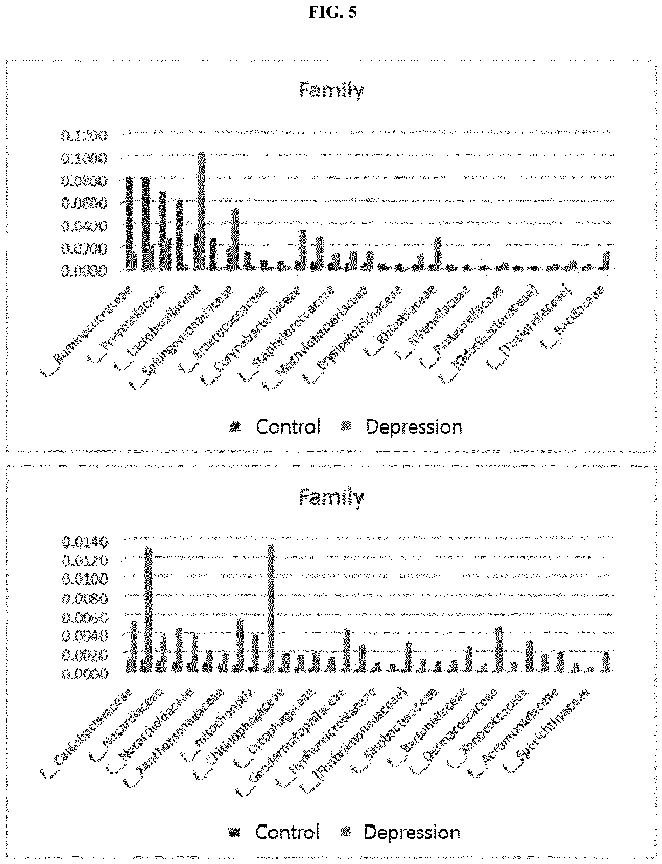

FIG. 5 is a result showing the distribution of bacteria-derived extracellular vesicles (EVs), which is significant in diagnostic performance at the family level by isolating bacteria-derived vesicles from urine of a patient with depression and a normal individual, and then performing a metagenomic analysis.

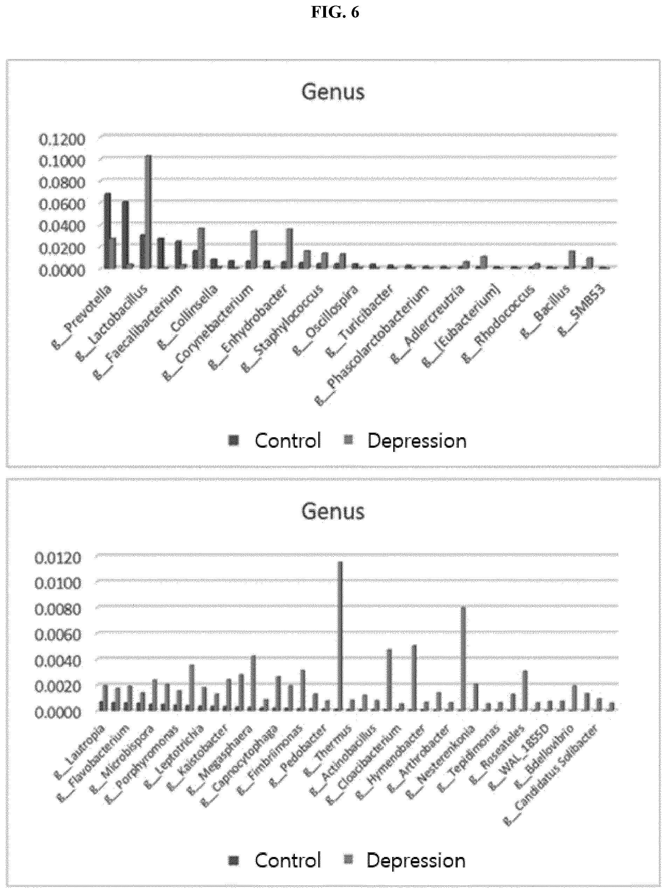

FIG. 6 is a result showing the distribution of bacteria-derived extracellular vesicles (EVs), which is significant in diagnostic performance at the genus level by isolating bacteria-derived vesicles from urine of a patient with depression and a normal individual, and then performing a metagenomic analysis.

### [Best Mode]

The present invention relates to a method of diagnosing depression through bacterial metagenomic analysis. The inventors of the present invention extracted genes from bacteria-derived extracellular vesicles using a subject-derived sample, performed metagenomic analysis thereon, and identified bacteria-derived extracellular vesicles capable of acting as a causative factor of depression.

Therefore, the present invention provides a method of providing information for diagnosing depression, the method comprising:
(a) extracting DNAs from extracellular vesicles isolated from subject samples;
(b) performing polymerase chain reaction (PCR) on the extracted DNA using a pair of primers comprising SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

The term "depression diagnosis" as used herein refers to determining whether a patient has a risk for depression, whether the risk for depression is relatively high, or whether depression has already occurred. The method of the present invention may be used to delay the onset of depression through special and appropriate care for a specific patient, which is a patient having a high risk for depression or prevent the onset of depression. In addition, the method may be clinically used to determine treatment by selecting the most appropriate treatment method through early diagnosis of depression.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, bacterial metagenomic analysis is performed using bacteria-derived extracellular vesicles isolated from, for example, serum.

In the present invention, the subject samples may be urine, but the present invention is not limited thereto.

In an embodiment of the present invention, metagenomic analysis is performed on the bacteria-derived extracellular vesicles, and bacteria-derived extracellular vesicles capable of acting as a cause of the onset of depression were actually identified by analysis at phylum, class, order, family, and genus levels.

More particularly, in one embodiment of the present invention, as a result of performing bacterial metagenomic analysis on extracellular vesicles present in subject-derived urine samples at a phylum level, the content of extracellular vesicles derived from bacteria belonging to the phylum *Verrucomicrobia,* the phylum *Bacteroidetes,* the phylum *Actinobacteria,* the phylum *Cyanobacteria,* the phylum *Thermi,* the phylum *Fusobacteria,* the phylum *Acidobacteria,* the phylum *Chloroflexi,* and the phylum *Armatimonadetes* was significantly different between depression patients and normal individuals (see Example 4).

More particularly, in one embodiment of the present invention, as a result of performing bacterial metagenomic analysis on extracellular vesicles present in subject-derived urine samples at a class level, the content of extracellular vesicles derived from bacteria belonging to the class *Erysipelotrichi,* the class *Verrucomicrobiae*, the class *Bacteroidia,* the class *Clostridia,* the class *Chloroplast,* the class *Actinobacteria,* the class *Alphaproteobacteria,* the class *Deltaproteobacteria,* the class *Saprospirae,* the class *Flavobacteriia,* the class *Cytophagia,* the class *Deinococci,* the class *Sphingobacteriia,* the class *Fusobacteriia*, the class *Fimbriimonadia,* the class TM7-1, the class *Pedosphaerae,* the class *Oscillatoriophycideae,* the class *Anaerolineae,* the class *Acidobacteria*-6, the class *Solibacteres,* the class *Nostocophycideae,* and the class iii1-8 was significantly different between depression patients and normal individuals (see Example 4).

More particularly, in one embodiment of the present invention, as a result of performing bacterial metagenomic analysis on extracellular vesicles present in subject-derived urine samples at an order level, the content of extracellular vesicles derived from bacteria belonging to the order *Turicibacterales,* the order *Erysipelotrichales,* the order *Verrucomicrobiales*, the order RF39, the order *Bacteroidales,* the order *Enterobacteriales,* the order *Clostridiales*, the order *Pasteurellales,* the order *Sphingomonadales,* the order *Bacillales,* the order *Xanthomonadales,* the order *Streptophyta,* the order *Rhodobacterales,* the order *Caulobacterales,* the order *Actinomycetales,* the order *Saprospirales,* the order *Rhizobiales,* the order *Flavobacteriales,* the order *Cytophagales,* the order *Myxococcales,* the order *Rickettsiales*, the order *Thermales,* the order *Rhodospirillales,* the order *Deinococcales,* the order *Sphingobacteriales,* the order *Fusobacteriales,* the order *Fimbriimonadales*, the order BD7-3, the order *Pedosphaerales,* the order *Aeromonadales,* the order *Chroococcales,* the order 1025, the order *Bdellovibrionales,* the order iii1-15, the order *Solibacterales*, the order PYR10d3, and the order DS-18 was significantly different between depression patients and normal individuals (see Example 4).

More particularly, in one embodiment of the present invention, as a result of performing bacterial metagenomic analysis on extracellular vesicles present in subject-derived urine samples at a family level, the content of extracellular vesicles derived from bacteria belonging to the family *Turicibacteraceae,* the family *Erysipelotrichaceae,* the family *Verrucomicrobiaceae,* the family *Rikenellaceae,* the family *Bacteroidaceae,* the family *Odoribacteraceae,* the family S24-7, the family *Clostridiaceae,* the family *Ruminococcaceae*, the family *Enterococcaceae,* the family *Paraprevotellaceae*, the family *Enterobacteriaceae,* the family *Leuconostocaceae,* the family *Porphyromonadaceae*, the family *Prevotellaceae,* the family *Pasteurellaceae,* the family *Actinomycetaceae,* the family *Burkholderiaceae,* the family *Xanthomonadaceae,* the family *Intrasporangiaceae,* the family *Staphylococcaceae,* the family *Sphingomonadaceae,* the family *Propionibacteriaceae,* the family *Nocardiaceae,* the family *Lactobacillaceae,* the family *Methylobacteriaceae,* the family *Rhodobacteraceae,* the family *Tissierellaceae,* the family *Acetobacteraceae,* the family *Caulobacteraceae,* the family *Nocardioidaceae*, the family *Hyphomicrobiaceae,* the family *Chitinophagaceae,* the family *Chromatiaceae,* the family *Flavobacteriaceae,* the family *Micrococcaceae,* the family *Sphingobacteriaceae,* the family *Corynebacteriaceae,* the family *Cytophagaceae,* the family *Weeksellaceae*, the family *Sinobacteraceae,* the family *Thermaceae,* the family *Deinococcaceae,* the family *Rhizobiaceae,* the family *Mycobacteriaceae,* the family *Comamonadaceae,* the family *Bacillaceae,* the family *Dermabacteraceae,* the family *Erythrobacteraceae,* the family *Geodermatophilaceae,* the family *Fimbriimonadaceae*, the family *Bartonellaceae,* the family *Leptotrichiaceae,* the family *Dermacoccaceae,* the family *Rhodospirillaceae,* the family *Aeromonadaceae*, the family Rs-045, the family *Xenococcaceae,* the family *Sporichthyaceae,* the family *Bdellovibrionaceae,* and the family *Solibacteraceae* was significantly different between depression patients and normal individuals (see Example 4).

More particularly, in one embodiment of the present invention, as a result of performing bacterial metagenomic analysis on extracellular vesicles present in subject-derived urine samples at a genus level, the content of extracellular vesicles derived from bacteria belonging to the genus *Butyricimonas,* the genus *Turicibacter,* the genus SMB53, the genus *Eubacterium,* the genus *Akkermansia*, the genus *Proteus,* the genus *Ruminococcus*, the genus *Parabacteroides,* the genus *Roseburia,* the genus *Bacteroides,* the genus *Phascolarctobacterium,* the genus *Sporosarcina,* the genus *Weissella,* the genus *Faecalibacterium,* the genus *Collinsella,* the genus *Oscillospira,* the genus *Prevotella,* the genus *Sphingomonas,* the genus *Finegoldia*, the genus *Chryseobacterium,* the genus *Lautropia,* the genus *Methylobacterium,* the genus *Propionibacterium,* the genus *Rhodococcus,* the genus *Flavobacterium,* the genus *Staphylococcus,* the genus *Lactobacillus,* the genus *Porphyromonas,* the genus *Peptoniphilus,* the genus *Adlercreutzia,* the genus *Erwinia,* the genus *Citrobacter,* the genus *Microbispora,* the genus *Corynebacterium,* the genus *Leptotrichia,* the genus *Pedobacter,* the genus *Enhydrobacter,* the genus *Cloacibacterium,* the genus *Paracoccus,* the genus *Kaistobacter,* the genus *Thermus*, the genus *Actinobacillus,* the genus *Deinococcus,* the genus *Anaerococcus,* the genus *Hymenobacter,* the genus *Achromobacter,* the genus *Mycobacterium,* the genus *Agrobacterium,* the genus *Brachybacterium,* the genus *Capnocytophaga*, the genus *Bacillus,* the genus *Arthrobacter,* the genus *Megasphaera*, the genus *Fimbriimonas,* the genus *Peptostreptococcus,* the genus *Modestobacter,* the genus *Tepidimonas*, the genus *Dermacoccus*, the genus *Janthinobacterium*, the genus *Wautersiella,* the genus *Novosphingobium,* the genus *Nesterenkonia,* the genus *Sneathia,* the genus WAL_1855D, the genus *Skermanella,* the genus *Shuttleworthia,* the genus *Roseateles,* the genus *Bdellovibrio,* the genus *Actinobaculum,* the genus *Candidatus Solibacter,* and the genus *Chroococcidiopsis* was significantly different between depression patients and normal individuals (see Example 4).

Through the results of the examples, it was confirmed that distribution variables of the identified bacteria and archaea-derived extracellular vesicles could be usefully used for the prediction of the onset of depression.

### [Modes of the Invention]

Hereinafter, the present invention will be described with reference to exemplary examples to aid in understanding of the present invention. However, these examples are provided only for illustrative purposes and are not intended to limit the scope of the present invention.

### [Examples]

### Example 1. Analysis of In Vivo Absorption, Distribution, and Excretion Patterns of Intestinal Bacteria and Bacteria-Derived Extracellular Vesicles

To evaluate whether intestinal bacteria and bacteria-derived vesicles are absorbed systemically through the mucosa, an experiment was performed using the following method. More particularly, 50 µg of each of intestinal bacteria and the intestinal bacteria-derived extracellular vesicles (EVs), labeled with fluorescence, were orally administered to the gastrointestinal tracts of mice, and fluorescence was measured at 0 h, and after 5 min, 3 h, 6 h, and 12 h. As a result of observing the entire images of mice, as illustrated in FIG. 1A, the bacteria were not systematically absorbed when administered, while the bacteria-derived EVs were systematically absorbed at 5 min after administration, and, at 3 h after administration, fluorescence was strongly observed in the bladder, from which it was confirmed that the EVs were excreted via the urinary system, and were present in the bodies up to 12 h after administration.

After intestinal bacteria and intestinal bacteria-derived extracellular vesicles were systematically absorbed, to evaluate a pattern of invasion of intestinal bacteria and the bacteria-derived EVs into various organs in the human body after being systematically absorbed, 50 µg of each of the bacteria and bacteria-derived EVs, labeled with fluorescence, were administered using the same method as that used above, and then, at 12 h after administration, blood, the heart, the lungs, the liver, the kidneys, the spleen, adipose tissue, and muscle were extracted from each mouse. As a result of observing fluorescence in the extracted tissues, as illustrated in FIG. 1B, it was confirmed that the intestinal bacteria were not absorbed into each organ, while the intestinal bacteria-derived EVs were distributed in the urine, heart, lungs, liver, kidneys, spleen, adipose tissue, and muscle.

### Example 2. Vesicle Isolation and DNA Extraction from Urine

To isolate vesicles and extract DNA, from urine, first, urine was added to a 10 ml tube and centrifuged at 3,500 x g and 4 °C for 10 min to precipitate a suspension, and only a supernatant was collected, which was then placed in a new 10 ml tube. The collected supernatant was filtered using a 0.22 µm filter to remove bacteria and impurities, and then placed in centrifugal filters (50 kD) and centrifuged at 1500 x g and 4 °C for 15 min to discard materials with a smaller size than 50 kD, and then concentrated to 10 ml. Once again, bacteria and impurities were removed therefrom using a 0.22 µm filter, and then the resulting concentrate was subjected to ultra-high speed centrifugation at 150,000 x g and 4 °C for 3 hours by using a Type 90ti rotor to remove a supernatant, and the agglomerated pellet was dissolved with phosphate-buffered saline (PBS), thereby obtaining vesicles.

100 µl of the extracellular vesicles isolated from the urine according to the above-described method was boiled at 100 °C to allow the internal DNA to come out of the lipid and then cooled on ice for 5 minutes. Next, the resulting vesicles were centrifuged at 10,000 x g and 4 °C for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below.

**[Table 1]**

| Primer | | Sequence | SEQ ID NO. |
|---|---|---|---|
| 16S rDNA | 16S_V3_F | 5'-TCGTCGGCAGCGTC AGATGTGTATAAGAG ACAGCCTACGGGNGG CWGCAG-3' | 1 |
| | 16S_V4_R | 5'-GTCTCGTGGGCTCG GAGATGTGTATAAGA GACAGGACTACHVGG GTATCTAATCC-3' | 2 |

### Example 3. Metagenomic Analysis Using DNA Extracted from Urine

DNA was extracted using the same method as that used in Example 2, and then PCR was performed thereon using 16S rDNA primers shown in Table 1 to amplify DNA, followed by sequencing (Illumina MiSeq sequencer). The results were output as standard flowgram format (SFF) files, and the SFF files were converted into sequence files (.fasta) and nucleotide quality score files using GS FLX software (v2.9), and then credit rating for reads was identified, and portions with a window (20 bps) average base call accuracy of less than 99% (Phred score <20) were removed. After removing the low-quality portions, only reads having a length of 300 bps or more were used (Sickle version 1.33), and, for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S DNA sequence databases (108,453 sequences) of BLASTN and GreenGenes.

### Example 4. Depression Diagnostic Model Based on Metagenomic Analysis of Bacteria-Derived EVs Isolated from Urine

EVs were isolated from urine samples of 20 depression patients and 21 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

As a result of analyzing bacteria-derived EVs in urine at a phylum level, a diagnostic model developed using bacteria belonging to the phylum *Verrucomicrobia,* the phylum *Bacteroidetes,* the phylum *Actinobacteria*, the phylum *Cyanobacteria,* the phylum *Thermi,* the phylum *Fusobacteria,* the phylum *Acidobacteria,* the phylum *Chloroflexi,* and the phylum *Armatimonadetes* as a biomarker exhibited significant diagnostic performance for depression (see Table 2 and FIG. 2).

**[Table 2]**

| | Control | | Depression | | t-test | | | | |
|---|---|---|---|---|---|---|---|---|---|
| Taxon | Mean | SD | Mean | SD | p-value | Ratio | AUC | sensitivity | specificity |
| p_Verrucomicrobia | 0.0269 | 0.0366 | 0.0017 | 0.0005 | 0.0048 | 0.06 | 0.96 | 0.91 | 1.00 |
| p_Bacteroidetes | 0.1514 | 0.0667 | 0.0514 | 0.0059 | 0.0000 | 0.34 | 0.96 | 0.91 | 1.00 |
| p_Actinobacteria | 0.0588 | 0.0305 | 0.1626 | 0.0144 | 0.0000 | 2.77 | 1.00 | 0.95 | 0.91 |
| p_Cyanobacteria | 0.0102 | 0.0134 | 0.0358 | 0.0050 | 0.0000 | 3.51 | 0.96 | 0.95 | 0.82 |
| p_Thermi | 0.0003 | 0.0006 | 0.0022 | 0.0006 | 0.0000 | 8.16 | 0.99 | 0.95 | 1.00 |
| p_Fusobacteria | 0.0011 | 0.0015 | 0.0149 | 0.0038 | 0.0000 | 13.17 | 1.00 | 1.00 | 1.00 |
| p_Acidobacteria | 0.0004 | 0.0015 | 0.0058 | 0.0025 | 0.0000 | 13.61 | 0.97 | 0.95 | 0.82 |
| p_ Chloroflexi | 0.0002 | 0.0003 | 0.0026 | 0.0019 | 0.0024 | 14.43 | 1.00 | 1.00 | 1.00 |
| p_Armatimonadetes | 0.0002 | 0.0004 | 0.0031 | 0.0011 | 0.0000 | 18.33 | 1.00 | 0.95 | 0.91 |

As a result of analyzing bacteria-derived EVs in urine at a class level, a diagnostic model developed using bacteria belonging to the class *Erysipelotrichi,* the class *Verrucomicrobiae*, the class *Bacteroidia,* the class *Clostridia,* the class *Chloroplast,* the class *Actinobacteria,* the class *Alphaproteobacteria,* the class *Deltaproteobacteria,* the class *Saprospirae,* the class *Flavobacteriia,* the class *Cytophagia,* the class *Deinococci,* the class *Sphingobacteriia,* the class *Fusobacteriia,* the class *Fimbriimonadia,* the class TM7-1, the class *Pedosphaerae,* the class *Oscillatoriophycideae,* the class *Anaerolineae,* the class *Acidobacteria*-6, the class *Solibacteres,* the class *Nostocophycideae,* and the class iii1-8 as a biomarker exhibited significant diagnostic performance for depression (see Table 3 and FIG. 3).

**[Table 3]**

| | Control | | Depression | | t-test | | | | |
|---|---|---|---|---|---|---|---|---|---|
| Taxon | Mean | SD | Mean | SD | p-value | Ratio | AUC | sensitivity | specificity |
| c_ Erysipelotrichi | 0.0040 | 0.0052 | 0.0001 | 0.0001 | 0.0023 | 0.02 | 0.98 | 0.91 | 0.91 |
| c_Verrucomicrobiae | 0.0268 | 0.0366 | 0.0005 | 0.0003 | 0.0036 | 0.02 | 0.98 | 0.95 | 1.00 |
| c_ Bacteroidia | 0.1482 | 0.0651 | 0.0338 | 0.0063 | 0.0000 | 0.23 | 0.98 | 0.95 | 1.00 |
| c_Clostridia | 0.2139 | 0.0555 | 0.0698 | 0.0081 | 0.0000 | 0.33 | 0.99 | 0.95 | 1.00 |
| c_Chloroplast | 0.0097 | 0.0135 | 0.0298 | 0.0027 | 0.0000 | 3.07 | 0.95 | 0.95 | 0.82 |
| c_ Actinobacteria | 0.0443 | 0.0230 | 0.1448 | 0.0121 | 0.0000 | 3.27 | 1.00 | 1.00 | 1.00 |
| c_Alphaproteobacteria | 0.0382 | 0.0328 | 0.1349 | 0.0089 | 0.0000 | 3.54 | 0.98 | 0.95 | 1.00 |
| c_Deltaproteobacteria | 0.0009 | 0.0011 | 0.0036 | 0.0006 | 0.0000 | 3.77 | 0.98 | 0.91 | 0.82 |
| c_Saprospirae | 0.0004 | 0.0010 | 0.0020 | 0.0008 | 0.0002 | 4.40 | 0.92 | 0.91 | 0.64 |
| c_ Flavobacteriia | 0.0018 | 0.0019 | 0.0102 | 0.0014 | 0.0000 | 5.64 | 1.00 | 1.00 | 1.00 |
| c_Cytophagia | 0.0004 | 0.0008 | 0.0022 | 0.0007 | 0.0000 | 5.76 | 0.93 | 0.91 | 0.73 |
| c_ Deinococci | 0.0003 | 0.0006 | 0.0022 | 0.0006 | 0.0000 | 8.16 | 0.99 | 0.95 | 1.00 |
| c_Sphingobacteriia | 0.0003 | 0.0005 | 0.0033 | 0.0023 | 0.0019 | 11.49 | 1.00 | 1.00 | 1.00 |
| c_ Fusobacteriia | 0.0011 | 0.0015 | 0.0149 | 0.0038 | 0.0000 | 13.17 | 1.00 | 1.00 | 1.00 |
| c_ Fimbriimonadia | 0.0002 | 0.0004 | 0.0031 | 0.0011 | 0.0000 | 18.33 | 1.00 | 0.95 | 0.91 |
| c_TM7-1 | 0.0000 | 0.0001 | 0.0008 | 0.0005 | 0.0010 | 20.50 | 0.99 | 0.95 | 0.91 |
| c_ Pedosphaerae | 0.0000 | 0.0001 | 0.0007 | 0.0006 | 0.0042 | 48.86 | 0.99 | 0.95 | 0.91 |
| c_Oscillatoriophycideae | 0.0000 | 0.0001 | 0.0033 | 0.0012 | 0.0000 | 106.00 | 1.00 | 1.00 | 1.00 |
| c_ Anaerolineae | 0.0000 | 0.0000 | 0.0005 | 0.0003 | 0.0004 | 121.68 | 1.00 | 1.00 | 1.00 |
| c_ Acidobacteria-6 | 0.0000 | 0.0000 | 0.0012 | 0.0008 | 0.0006 | 276.36 | 1.00 | 1.00 | 1.00 |
| c_Solibacteres | 0.0000 | 0.0000 | 0.0025 | 0.0014 | 0.0002 | 570.67 | 1.00 | 1.00 | 1.00 |
| c_Nostocophycideae | 0.0000 | 0.0000 | 0.0006 | 0.0006 | 0.0002 | >100 | 1.00 | 1.00 | 1.00 |
| c_iii1-8 | 0.0000 | 0.0000 | 0.0005 | 0.0006 | 0.0007 | >100 | 1.00 | 1.00 | 1.00 |

As a result of analyzing bacteria-derived EVs in urine at an order level, a diagnostic model developed using bacteria belonging to the order *Turicibacterales,* the order *Erysipelotrichales,* the order *Verrucomicrobiales*, the order RF39, the order *Bacteroidales,* the order *Enterobacteriales,* the order *Clostridiales,* the order *Pasteurellales,* the order *Sphingomonadales,* the order *Bacillales,* the order *Xanthomonadales,* the order *Streptophyta,* the order *Rhodobacterales,* the order *Caulobacterales,* the order *Actinomycetales,* the order *Saprospirales,* the order *Rhizobiales,* the order *Flavobacteriales,* the order *Cytophagales,* the order *Myxococcales,* the order *Rickettsiales,* the order *Thermales,* the order *Rhodospirillales,* the order *Deinococcales,* the order *Sphingobacteriales,* the order *Fusobacteriales,* the order *Fimbriimonadales*, the order BD7-3, the order *Pedosphaerales,* the order *Aeromonadales,* the order *Chroococcales,* the order 1025, the order *Bdellovibrionales,* the order iii1-15, the order *Solibacterales,* the order PYR10d3, and the order DS-18 as a biomarker exhibited significant diagnostic performance for depression (see Table 4 and FIG. 4).

**[Table 4]**

| | Control | | Depression | | t-test | | | | |
|---|---|---|---|---|---|---|---|---|---|
| Taxon | Mean | SD | Mean | SD | p-value | Ratio | AUC | sensitivity | specificity |
| o_ Turicibacterales | 0.0023 | 0.0037 | 0.0000 | 0.0001 | 0.0091 | 0.01 | 0.85 | 0.77 | 0.64 |
| o_ Erysipelotrichales | 0.0040 | 0.0052 | 0.0001 | 0.0001 | 0.0023 | 0.02 | 0.98 | 0.91 | 0.91 |
| o_ Verrucomicrobiales | 0.0268 | 0.0366 | 0.0005 | 0.0003 | 0.0036 | 0.02 | 0.98 | 0.95 | 1.00 |
| o_RF39 | 0.0069 | 0.0102 | 0.0006 | 0.0008 | 0.0100 | 0.09 | 0.93 | 0.86 | 0.64 |
| o_ Bacteroidales | 0.1482 | 0.0651 | 0.0338 | 0.0063 | 0.0000 | 0.23 | 0.98 | 0.95 | 1.00 |
| o_Enterobacteriales | 0.0808 | 0.0532 | 0.0212 | 0.0032 | 0.0000 | 0.26 | 0.88 | 0.82 | 1.00 |
| o_Clostridiales | 0.2137 | 0.0555 | 0.0697 | 0.0081 | 0.0000 | 0.33 | 0.99 | 0.95 | 1.00 |
| o_Pasteurellales | 0.0027 | 0.0024 | 0.0053 | 0.0015 | 0.0022 | 2.00 | 0.81 | 0.82 | 0.45 |
| o_Sphingomonadales | 0.0193 | 0.0230 | 0.0547 | 0.0047 | 0.0000 | 2.83 | 0.95 | 0.91 | 1.00 |
| o_ Bacillales | 0.0150 | 0.0097 | 0.0458 | 0.0091 | 0.0000 | 3.05 | 0.98 | 0.95 | 0.91 |
| o_Xanthomonadales | 0.0010 | 0.0013 | 0.0030 | 0.0009 | 0.0001 | 3.07 | 0.92 | 0.91 | 0.73 |
| ο_Streptophyta | 0.0093 | 0.0130 | 0.0295 | 0.0027 | 0.0000 | 3.18 | 0.95 | 0.95 | 0.82 |
| o_ Rhodobacterales | 0.0037 | 0.0078 | 0.0132 | 0.0026 | 0.0000 | 3.57 | 0.94 | 0.91 | 0.73 |
| o_Caulobacterales | 0.0013 | 0.0018 | 0.0054 | 0.0014 | 0.0000 | 4.02 | 1.00 | 1.00 | 1.00 |
| o_Actinomycetales | 0.0281 | 0.0167 | 0.1140 | 0.0077 | 0.0000 | 4.05 | 1.00 | 1.00 | 1.00 |
| o_Saprospirales | 0.0004 | 0.0010 | 0.0020 | 0.0008 | 0.0002 | 4.40 | 0.92 | 0.91 | 0.64 |
| o_ Rhizobiales | 0.0120 | 0.0112 | 0.0530 | 0.0077 | 0.0000 | 4.42 | 1.00 | 0.95 | 0.91 |
| o_Flavobacteriales | 0.0018 | 0.0019 | 0.0102 | 0.0014 | 0.0000 | 5.64 | 1.00 | 1.00 | 1.00 |
| o_Cytophagales | 0.0004 | 0.0008 | 0.0022 | 0.0007 | 0.0000 | 5.76 | 0.93 | 0.91 | 0.73 |
| o_Myxococcales | 0.0002 | 0.0004 | 0.0014 | 0.0006 | 0.0000 | 6.56 | 0.97 | 0.91 | 0.82 |
| o_ Rickettsiales | 0.0006 | 0.0011 | 0.0039 | 0.0014 | 0.0000 | 6.57 | 0.98 | 0.95 | 0.91 |
| o_ Thermales | 0.0001 | 0.0002 | 0.0008 | 0.0005 | 0.0006 | 7.84 | 0.95 | 0.91 | 0.82 |
| o_Rhodospirillales | 0.0005 | 0.0015 | 0.0037 | 0.0013 | 0.0000 | 7.95 | 0.96 | 0.95 | 0.73 |
| o_ Deinococcales | 0.0002 | 0.0006 | 0.0014 | 0.0003 | 0.0000 | 8.36 | 0.95 | 0.95 | 0.91 |
| o_Sphingobacteriales | 0.0003 | 0.0005 | 0.0033 | 0.0023 | 0.0019 | 11.49 | 1.00 | 1.00 | 1.00 |
| o_Fusobacteriales | 0.0011 | 0.0015 | 0.0149 | 0.0038 | 0.0000 | 13.17 | 1.00 | 1.00 | 1.00 |
| o_Fimbriimonadales | 0.0002 | 0.0004 | 0.0031 | 0.0011 | 0.0000 | 18.33 | 1.00 | 0.95 | 0.91 |
| o_BD7-3 | 0.0000 | 0.0001 | 0.0007 | 0.0007 | 0.0083 | 35.43 | 0.99 | 1.00 | 0.91 |
| o_ Pedosphaerales | 0.0000 | 0.0001 | 0.0007 | 0.0006 | 0.0042 | 48.86 | 0.99 | 0.95 | 0.91 |
| o_ Aeromonadales | 0.0000 | 0.0001 | 0.0020 | 0.0009 | 0.0000 | 55.39 | 1.00 | 1.00 | 1.00 |
| o_Chroococcales | 0.0000 | 0.0001 | 0.0033 | 0.0012 | 0.0000 | 105.88 | 1.00 | 1.00 | 1.00 |
| o_I025 | 0.0000 | 0.0000 | 0.0010 | 0.0006 | 0.0003 | 107.24 | 1.00 | 1.00 | 1.00 |
| o_Bdellovibrionales | 0.0000 | 0.0001 | 0.0020 | 0.0008 | 0.0000 | 131.35 | 1.00 | 1.00 | 1.00 |
| o_iii1-15 | 0.0000 | 0.0000 | 0.0012 | 0.0008 | 0.0010 | 263.10 | 0.97 | 1.00 | 0.91 |
| o_Solibacterales | 0.0000 | 0.0000 | 0.0025 | 0.0014 | 0.0002 | 570.67 | 1.00 | 1.00 | 1.00 |
| o_PYR10d3 | 0.0000 | 0.0000 | 0.0011 | 0.0004 | 0.0000 | >100 | 1.00 | 1.00 | 1.00 |
| o_DS-18 | 0.0000 | 0.0000 | 0.0005 | 0.0006 | 0.0007 | >100 | 1.00 | 1.00 | 1.00 |

As a result of analyzing bacteria-derived EVs in urine at a family level, a diagnostic model developed using bacteria belonging to the family *Turicibacteraceae,* the family *Erysipelotrichaceae,* the family *Verrucomicrobiaceae,* the family *Rikenellaceae,* the family *Bacteroidaceae,* the family *Odoribacteraceae,* the family S24-7, the family *Clostridiaceae,* the family *Ruminococcaceae*, the family *Enterococcaceae,* the family *Paraprevotellaceae,* the family *Enterobacteriaceae,* the family *Leuconostocaceae,* the family *Porphyromonadaceae*, the family *Prevotellaceae,* the family *Pasteurellaceae,* the family *Actinomycetaceae,* the family *Burkholderiaceae,* the family *Xanthomonadaceae*, the family *Intrasporangiaceae,* the family *Staphylococcaceae,* the family *Sphingomonadaceae,* the family *Propionibacteriaceae,* the family *Nocardiaceae,* the family *Lactobacillaceae,* the family *Methylobacteriaceae,* the family *Rhodobacteraceae,* the family *Tissierellaceae*, the family *Acetobacteraceae,* the family *Caulobacteraceae,* the family *Nocardioidaceae,* the family *Hyphomicrobiaceae*, the family *Chitinophagaceae,* the family *Chromatiaceae,* the family *Flavobacteriaceae,* the family *Micrococcaceae,* the family *Sphingobacteriaceae,* the family *Corynebacteriaceae,* the family *Cytophagaceae,* the family *Weeksellaceae*, the family *Sinobacteraceae,* the family *Thermaceae,* the family *Deinococcaceae,* the family *Rhizobiaceae,* the family *Mycobacteriaceae,* the family *Comamonadaceae,* the family *Bacillaceae,* the family *Dermabacteraceae,* the family *Erythrobacteraceae,* the family *Geodermatophilaceae,* the family *Fimbriimonadaceae,* the family *Bartonellaceae,* the family *Leptotrichiaceae,* the family *Dermacoccaceae,* the family *Rhodospirillaceae,* the family *Aeromonadaceae,* the family Rs-045, the family *Xenococcaceae,* the family *Sporichthyaceae,* the family *Bdellovibrionaceae,* and the family *Solibacteraceae* as a biomarker exhibited significant diagnostic performance for depression (see Table 5 and FIG. 5).

**[Table 5]**

| | Control | | Depression | | t-test | | | | |
|---|---|---|---|---|---|---|---|---|---|
| Taxon | Mean | SD | Mean | SD | p-value | Ratio | AUC | sensitivity | specificity |
| f_Turicibacteraceae | 0.0023 | 0.0037 | 0.0000 | 0.0001 | 0.0091 | 0.01 | 0.85 | 0.77 | 0.64 |
| f_Erysipelotrichaceae | 0.0040 | 0.0052 | 0.0001 | 0.0001 | 0.0023 | 0.02 | 0.98 | 0.91 | 0.91 |
| f_Verrucomicrobiaceae | 0.0268 | 0.0366 | 0.0005 | 0.0003 | 0.0036 | 0.02 | 0.98 | 0.95 | 1.00 |
| f_Rikenellaceae | 0.0031 | 0.0030 | 0.0002 | 0.0002 | 0.0002 | 0.05 | 0.91 | 0.82 | 0.91 |
| f_ Bacteroidaceae | 0.0607 | 0.0416 | 0.0034 | 0.0010 | 0.0000 | 0.06 | 0.98 | 0.95 | 1.00 |
| f_ Odoribacteraceae | 0.0021 | 0.0027 | 0.0001 | 0.0001 | 0.0035 | 0.06 | 0.88 | 0.86 | 0.73 |
| f_S24-7 | 0.0035 | 0.0045 | 0.0004 | 0.0002 | 0.0056 | 0.11 | 0.84 | 0.86 | 0.73 |
| f_ Clostridiaceae | 0.0152 | 0.0108 | 0.0021 | 0.0006 | 0.0000 | 0.14 | 0.96 | 0.91 | 0.82 |
| f_Ruminococcaceae | 0.0819 | 0.0363 | 0.0152 | 0.0029 | 0.0000 | 0.19 | 0.99 | 0.95 | 0.91 |
| f_Enterococcaceae | 0.0075 | 0.0087 | 0.0014 | 0.0007 | 0.0042 | 0.19 | 0.88 | 0.82 | 0.73 |
| f_Paraprevotellaceae | 0.0027 | 0.0018 | 0.0007 | 0.0004 | 0.0000 | 0.25 | 0.86 | 0.82 | 0.64 |
| f_Enterobacteriaceae | 0.0808 | 0.0532 | 0.0212 | 0.0032 | 0.0000 | 0.26 | 0.88 | 0.82 | 1.00 |
| f_Leuconostocaceae | 0.0045 | 0.0032 | 0.0013 | 0.0006 | 0.0002 | 0.30 | 0.84 | 0.82 | 0.64 |
| f_Porphyromonadaceae | 0.0070 | 0.0065 | 0.0022 | 0.0009 | 0.0026 | 0.31 | 0.79 | 0.77 | 0.55 |
| f_Prevotellaceae | 0.0679 | 0.0620 | 0.0267 | 0.0056 | 0.0063 | 0.39 | 0.79 | 0.82 | 0.55 |
| f_Pasteurellaceae | 0.0027 | 0.0024 | 0.0053 | 0.0015 | 0.0022 | 2.00 | 0.81 | 0.82 | 0.45 |
| f_Actinomycetaceae | 0.0021 | 0.0030 | 0.0042 | 0.0010 | 0.0062 | 2.03 | 0.88 | 0.95 | 0.55 |
| f_Burkholderiaceae | 0.0010 | 0.0010 | 0.0022 | 0.0013 | 0.0074 | 2.26 | 0.78 | 0.86 | 0.36 |
| f_Xanthomonadaceae | 0.0008 | 0.0013 | 0.0019 | 0.0006 | 0.0027 | 2.29 | 0.87 | 0.91 | 0.36 |
| f_Intrasporangiaceae | 0.0015 | 0.0032 | 0.0039 | 0.0006 | 0.0038 | 2.53 | 0.90 | 0.91 | 0.55 |
| f_ Staphylococcaceae | 0.0050 | 0.0041 | 0.0136 | 0.0027 | 0.0000 | 2.74 | 0.98 | 0.91 | 0.91 |
| f_ Sphingomonadaceae | 0.0192 | 0.0231 | 0.0535 | 0.0048 | 0.0000 | 2.79 | 0.95 | 0.91 | 1.00 |
| f_Propionibacteriaceae | 0.0048 | 0.0039 | 0.0155 | 0.0035 | 0.0000 | 3.24 | 0.97 | 0.91 | 0.82 |
| f_Nocardiaceae | 0.0012 | 0.0025 | 0.0039 | 0.0015 | 0.0031 | 3.25 | 0.87 | 0.91 | 0.55 |
| f_Lactobacillaceae | 0.0313 | 0.0367 | 0.1029 | 0.0245 | 0.0000 | 3.29 | 0.95 | 0.95 | 0.91 |
| f_Methylobacteriaceae | 0.0047 | 0.0050 | 0.0159 | 0.0029 | 0.0000 | 3.36 | 0.97 | 0.95 | 0.91 |
| f_Rhodobacteraceae | 0.0037 | 0.0078 | 0.0132 | 0.0026 | 0.0000 | 3.56 | 0.93 | 0.91 | 0.73 |
| f_Tissierellaceae | 0.0019 | 0.0023 | 0.0071 | 0.0017 | 0.0000 | 3.81 | 0.97 | 0.91 | 0.91 |
| f_Acetobacteraceae | 0.0004 | 0.0015 | 0.0017 | 0.0007 | 0.0042 | 3.88 | 0.90 | 0.95 | 0.64 |
| f_ Caulobacteraceae | 0.0013 | 0.0018 | 0.0054 | 0.0014 | 0.0000 | 4.02 | 1.00 | 1.00 | 1.00 |
| f_Nocardioidaceae | 0.0010 | 0.0017 | 0.0039 | 0.0013 | 0.0000 | 4.04 | 0.92 | 0.91 | 0.82 |
| f_Hyphomicrobiaceae | 0.0002 | 0.0005 | 0.0010 | 0.0007 | 0.0026 | 4.27 | 0.88 | 0.91 | 0.55 |
| f_ Chitinophagaceae | 0.0004 | 0.0010 | 0.0019 | 0.0008 | 0.0002 | 4.32 | 0.92 | 0.91 | 0.64 |
| f_ Chromatiaceae | 0.0002 | 0.0006 | 0.0008 | 0.0005 | 0.0058 | 4.46 | 0.88 | 0.91 | 0.45 |
| f_Flavobacteriaceae | 0.0010 | 0.0018 | 0.0046 | 0.0011 | 0.0000 | 4.58 | 0.95 | 0.91 | 0.82 |
| f_Micrococcaceae | 0.0058 | 0.0067 | 0.0281 | 0.0055 | 0.0000 | 4.82 | 0.98 | 0.95 | 1.00 |
| f_ Sphingobacteriaceae | 0.0003 | 0.0005 | 0.0015 | 0.0007 | 0.0000 | 5.11 | 0.91 | 0.86 | 0.73 |
| f_ Corynebacteriaceae | 0.0064 | 0.0052 | 0.0335 | 0.0061 | 0.0000 | 5.24 | 1.00 | 1.00 | 1.00 |
| f_ Cytophagaceae | 0.0004 | 0.0008 | 0.0021 | 0.0007 | 0.0000 | 5.50 | 0.93 | 0.91 | 0.82 |
| f_Weeksellaceae | 0.0008 | 0.0012 | 0.0056 | 0.0011 | 0.0000 | 6.96 | 1.00 | 1.00 | 1.00 |
| f_ Sinobacteraceae | 0.0001 | 0.0006 | 0.0011 | 0.0008 | 0.0013 | 7.51 | 0.96 | 0.95 | 0.45 |
| f_Thermaceae | 0.0001 | 0.0002 | 0.0008 | 0.0005 | 0.0006 | 7.84 | 0.95 | 0.91 | 0.82 |
| f_Deinococcaceae | 0.0002 | 0.0006 | 0.0013 | 0.0003 | 0.0000 | 7.99 | 0.95 | 0.95 | 0.91 |
| f_Rhizobiaceae | 0.0035 | 0.0034 | 0.0283 | 0.0048 | 0.0000 | 8.17 | 1.00 | 1.00 | 1.00 |
| f_Mycobacteriaceae | 0.0003 | 0.0008 | 0.0028 | 0.0017 | 0.0009 | 10.04 | 0.98 | 0.95 | 0.82 |
| f_ Comamonadaceae | 0.0013 | 0.0015 | 0.0131 | 0.0047 | 0.0000 | 10.26 | 1.00 | 1.00 | 1.00 |
| f_Bacillaceae | 0.0014 | 0.0015 | 0.0157 | 0.0080 | 0.0002 | 11.61 | 1.00 | 1.00 | 1.00 |
| f_Dermabacteraceae | 0.0001 | 0.0002 | 0.0013 | 0.0010 | 0.0032 | 12.37 | 0.98 | 0.95 | 0.73 |
| f_Erythrobacteraceae | 0.0001 | 0.0002 | 0.0009 | 0.0005 | 0.0001 | 12.56 | 1.00 | 1.00 | 1.00 |
| f_Geodermatophilaceae | 0.0003 | 0.0008 | 0.0044 | 0.0014 | 0.0000 | 15.70 | 1.00 | 1.00 | 1.00 |
| f_Fimbriimonadaceae | 0.0002 | 0.0004 | 0.0031 | 0.0011 | 0.0000 | 18.33 | 1.00 | 0.95 | 0.91 |
| f_Bartonellaceae | 0.0001 | 0.0003 | 0.0027 | 0.0009 | 0.0000 | 26.23 | 1.00 | 1.00 | 1.00 |
| f_Leptotrichiaceae | 0.0004 | 0.0007 | 0.0133 | 0.0037 | 0.0000 | 29.87 | 1.00 | 1.00 | 1.00 |
| f_Dermacoccaceae | 0.0001 | 0.0002 | 0.0047 | 0.0005 | 0.0000 | 50.52 | 1.00 | 1.00 | 1.00 |
| f_Rhodospirillaceae | 0.0000 | 0.0001 | 0.0018 | 0.0007 | 0.0000 | 69.14 | 1.00 | 1.00 | 1.00 |
| f_Aeromonadaceae | 0.0000 | 0.0001 | 0.0020 | 0.0009 | 0.0000 | 96.40 | 1.00 | 1.00 | 1.00 |
| f_Rs-045 | 0.0000 | 0.0000 | 0.0009 | 0.0005 | 0.0001 | 99.47 | 1.00 | 1.00 | 1.00 |
| f_Xenococcaceae | 0.0000 | 0.0001 | 0.0033 | 0.0012 | 0.0000 | 105.88 | 1.00 | 1.00 | 1.00 |
| f_ Sporichthyaceae | 0.0000 | 0.0000 | 0.0005 | 0.0004 | 0.0022 | 142.51 | 0.98 | 1.00 | 0.91 |
| f_Bdellovibrionaceae | 0.0000 | 0.0000 | 0.0019 | 0.0008 | 0.0000 | 871.83 | 1.00 | 1.00 | 1.00 |
| f_ Solibacteraceae | 0.0000 | 0.0000 | 0.0009 | 0.0011 | 0.0008 | >100 | 1.00 | 1.00 | 1.00 |

As a result of analyzing bacteria-derived EVs in urine at a genus level, a diagnostic model developed using bacteria belonging to the genus *Butyricimonas,* the genus *Turicibacter,* the genus SMB53, the genus *Eubacterium,* the genus *Akkermansia,* the genus *Proteus,* the genus *Ruminococcus*, the genus *Parabacteroides,* the genus *Roseburia,* the genus *Bacteroides,* the genus *Phascolarctobacterium,* the genus *Sporosarcina,* the genus *Weissella,* the genus *Faecalibacterium,* the genus *Collinsella,* the genus *Oscillospira,* the genus *Prevotella,* the genus *Sphingomonas,* the genus *Finegoldia*, the genus *Chryseobacterium,* the genus *Lautropia,* the genus *Methylobacterium,* the genus *Propionibacterium,* the genus *Rhodococcus,* the genus *Flavobacterium,* the genus *Staphylococcus,* the genus *Lactobacillus,* the genus *Porphyromonas,* the genus *Peptoniphilus,* the genus *Adlercreutzia,* the genus *Erwinia,* the genus *Citrobacter,* the genus *Microbispora,* the genus *Corynebacterium,* the genus *Leptotrichia,* the genus *Pedobacter,* the genus *Enhydrobacter*, the genus *Cloacibacterium,* the genus *Paracoccus,* the genus *Kaistobacter,* the genus *Thermus*, the genus *Actinobacillus,* the genus *Deinococcus,* the genus *Anaerococcus,* the genus *Hymenobacter,* the genus *Achromobacter,* the genus *Mycobacterium,* the genus *Agrobacterium,* the genus *Brachybacterium*, the genus *Capnocytophaga*, the genus *Bacillus,* the genus *Arthrobacter,* the genus *Megasphaera*, the genus *Fimbriimonas,* the genus *Peptostreptococcus,* the genus *Modestobacter,* the genus *Tepidimonas,* the genus *Dermacoccus,* the genus *Janthinobacterium,* the genus *Wautersiella*, the genus *Novosphingobium,* the genus *Nesterenkonia,* the genus *Sneathia*, the genus WAL_1855D, the genus *Skermanella,* the genus *Shuttleworthia,* the genus *Roseateles,* the genus *Bdellovibrio,* the genus *Actinobaculum,* the genus *Candidatus Solibacter,* and the genus *Chroococcidiopsis* as a biomarker exhibited significant diagnostic performance for depression (see Table 6 and FIG. 6).

**[Table 6]**

| | Control | | Depression | | t-test | | | | |
|---|---|---|---|---|---|---|---|---|---|
| Taxon | Mean | SD | Mean | SD | p-value | Ratio | AUC | sensitivity | specificity |
| g_Butyricimonas | 0.0015 | 0.0018 | 0.0000 | 0.0000 | 0.0013 | 0.01 | 0.90 | 0.86 | 0.73 |
| g_Turicibacter | 0.0023 | 0.0037 | 0.0000 | 0.0001 | 0.0091 | 0.01 | 0.85 | 0.77 | 0.64 |
| g_SMB53 | 0.0007 | 0.0008 | 0.0000 | 0.0000 | 0.0004 | 0.01 | 0.97 | 0.91 | 0.82 |
| g_ Eubacterium | 0.0013 | 0.0018 | 0.0000 | 0.0001 | 0.0052 | 0.01 | 0.91 | 0.95 | 0.64 |
| g_ Akkermansia | 0.0267 | 0.0366 | 0.0004 | 0.0002 | 0.0034 | 0.01 | 0.98 | 0.95 | 1.00 |
| g_ Proteus | 0.0063 | 0.0082 | 0.0002 | 0.0001 | 0.0028 | 0.03 | 0.88 | 0.82 | 1.00 |
| g_Ruminococcus | 0.0034 | 0.0035 | 0.0002 | 0.0001 | 0.0004 | 0.04 | 0.91 | 0.82 | 0.73 |
| g_ Parabacteroides | 0.0066 | 0.0062 | 0.0003 | 0.0002 | 0.0001 | 0.05 | 0.96 | 0.91 | 0.91 |
| g_ Roseburia | 0.0011 | 0.0015 | 0.0001 | 0.0001 | 0.0043 | 0.05 | 0.91 | 0.86 | 0.82 |
| g_ Bacteroides | 0.0606 | 0.0414 | 0.0034 | 0.0010 | 0.0000 | 0.06 | 0.98 | 0.95 | 1.00 |
| g_Phascolarctobacterium | 0.0016 | 0.0021 | 0.0001 | 0.0002 | 0.0036 | 0.07 | 0.84 | 0.95 | 0.55 |
| g_Sporosarcina | 0.0013 | 0.0018 | 0.0001 | 0.0001 | 0.0088 | 0.09 | 0.73 | 0.95 | 0.45 |
| g_Weissella | 0.0023 | 0.0019 | 0.0003 | 0.0002 | 0.0001 | 0.13 | 0.91 | 0.86 | 0.73 |
| g_Faecalibacterium | 0.0241 | 0.0233 | 0.0031 | 0.0012 | 0.0005 | 0.13 | 0.93 | 0.77 | 0.73 |
| g_Collinsella | 0.0080 | 0.0103 | 0.0015 | 0.0008 | 0.0083 | 0.18 | 0.86 | 0.82 | 0.64 |
| g_Oscillospira | 0.0037 | 0.0035 | 0.0008 | 0.0013 | 0.0022 | 0.21 | 0.84 | 0.86 | 0.64 |
| g_ Prevotella | 0.0679 | 0.0620 | 0.0267 | 0.0056 | 0.0063 | 0.39 | 0.79 | 0.82 | 0.55 |
| g_Sphingomonas | 0.0157 | 0.0230 | 0.0359 | 0.0032 | 0.0007 | 2.29 | 0.89 | 0.86 | 0.55 |
| g_ Finegoldia | 0.0006 | 0.0011 | 0.0014 | 0.0005 | 0.0090 | 2.41 | 0.90 | 0.91 | 0.27 |
| g_Chryseobacterium | 0.0006 | 0.0011 | 0.0017 | 0.0008 | 0.0082 | 2.81 | 0.87 | 0.82 | 0.55 |
| g_ Lautropia | 0.0007 | 0.0010 | 0.0020 | 0.0013 | 0.0039 | 2.87 | 0.80 | 0.86 | 0.36 |
| g Methyl ob acterium | 0.0040 | 0.0049 | 0.0127 | 0.0022 | 0.0000 | 3.17 | 0.93 | 0.91 | 0.82 |
| g_Propionibacterium | 0.0048 | 0.0039 | 0.0155 | 0.0035 | 0.0000 | 3.23 | 0.97 | 0.91 | 0.82 |
| g_ Rhodococcus | 0.0012 | 0.0025 | 0.0039 | 0.0015 | 0.0034 | 3.23 | 0.87 | 0.91 | 0.55 |
| g_Flavobacterium | 0.0006 | 0.0014 | 0.0019 | 0.0006 | 0.0008 | 3.25 | 0.87 | 0.86 | 0.55 |
| g_Staphylococcus | 0.0040 | 0.0035 | 0.0133 | 0.0027 | 0.0000 | 3.29 | 0.99 | 0.95 | 0.91 |
| g_Lactobacillus | 0.0299 | 0.0370 | 0.1024 | 0.0243 | 0.0000 | 3.42 | 0.95 | 0.95 | 0.91 |
| g_ Porphyromonas | 0.0004 | 0.0007 | 0.0015 | 0.0009 | 0.0008 | 3.68 | 0.87 | 0.91 | 0.55 |
| g_Peptoniphilus | 0.0002 | 0.0004 | 0.0008 | 0.0005 | 0.0004 | 3.75 | 0.88 | 0.91 | 0.55 |
| g_ Adlercreutzia | 0.0015 | 0.0025 | 0.0057 | 0.0021 | 0.0001 | 3.90 | 0.91 | 0.86 | 0.73 |
| g_ Erwinia | 0.0003 | 0.0005 | 0.0013 | 0.0004 | 0.0000 | 4.02 | 0.94 | 0.91 | 0.82 |
| g_Citrobacter | 0.0005 | 0.0006 | 0.0020 | 0.0005 | 0.0000 | 4.24 | 0.97 | 0.95 | 0.82 |
| g_ Microbispora | 0.0005 | 0.0007 | 0.0024 | 0.0009 | 0.0000 | 4.92 | 0.98 | 0.91 | 0.82 |
| g_Corynebacterium | 0.0064 | 0.0052 | 0.0335 | 0.0061 | 0.0000 | 5.24 | 1.00 | 1.00 | 1.00 |
| g_ Leptotrichia | 0.0003 | 0.0005 | 0.0018 | 0.0006 | 0.0000 | 5.48 | 0.96 | 0.95 | 0.82 |
| g_Pedobacter | 0.0001 | 0.0003 | 0.0007 | 0.0005 | 0.0052 | 5.85 | 0.92 | 0.91 | 0.55 |
| g_ Enhydrobacter | 0.0056 | 0.0050 | 0.0353 | 0.0070 | 0.0000 | 6.28 | 1.00 | 1.00 | 1.00 |
| g_Cloacibacterium | 0.0001 | 0.0002 | 0.0005 | 0.0003 | 0.0009 | 6.63 | 0.91 | 0.91 | 0.64 |
| g_ Paracoccus | 0.0014 | 0.0021 | 0.0105 | 0.0018 | 0.0000 | 7.36 | 1.00 | 0.95 | 0.91 |
| g_ Kaistobacter | 0.0003 | 0.0009 | 0.0024 | 0.0009 | 0.0000 | 7.76 | 0.96 | 0.91 | 0.91 |
| g_Thermus | 0.0001 | 0.0002 | 0.0008 | 0.0005 | 0.0006 | 7.84 | 0.95 | 0.91 | 0.82 |
| g_ Actinobacillus | 0.0001 | 0.0001 | 0.0008 | 0.0005 | 0.0011 | 7.92 | 0.95 | 0.91 | 0.82 |
| g_ Deinococcus | 0.0002 | 0.0006 | 0.0013 | 0.0003 | 0.0000 | 7.95 | 0.95 | 0.95 | 0.91 |
| g_ Anaerococcus | 0.0004 | 0.0007 | 0.0035 | 0.0015 | 0.0000 | 9.31 | 1.00 | 1.00 | 1.00 |
| g_Hymenobacter | 0.0001 | 0.0002 | 0.0006 | 0.0003 | 0.0000 | 9.52 | 0.87 | 0.95 | 0.91 |
| g_Achromobacter | 0.0002 | 0.0006 | 0.0019 | 0.0008 | 0.0000 | 9.96 | 0.97 | 0.95 | 0.73 |
| g_ Mycobacterium | 0.0003 | 0.0008 | 0.0028 | 0.0017 | 0.0009 | 10.04 | 0.98 | 0.95 | 0.82 |
| g_ Agrobacterium | 0.0008 | 0.0017 | 0.0093 | 0.0024 | 0.0000 | 11.35 | 1.00 | 1.00 | 1.00 |
| g_ Brachybacterium | 0.0001 | 0.0002 | 0.0012 | 0.0009 | 0.0037 | 12.07 | 0.97 | 0.95 | 0.73 |
| g_Capnocytophaga | 0.0002 | 0.0005 | 0.0026 | 0.0010 | 0.0000 | 12.59 | 0.99 | 0.95 | 0.91 |
| g_ Bacillus | 0.0011 | 0.0013 | 0.0151 | 0.0080 | 0.0002 | 14.08 | 1.00 | 1.00 | 1.00 |
| g_ Arthrobacter | 0.0000 | 0.0001 | 0.0006 | 0.0004 | 0.0009 | 16.55 | 0.99 | 0.95 | 0.91 |
| g_Megasphaera | 0.0002 | 0.0007 | 0.0042 | 0.0010 | 0.0000 | 17.22 | 1.00 | 1.00 | 1.00 |
| g_ Fimbriimonas | 0.0002 | 0.0004 | 0.0031 | 0.0011 | 0.0000 | 18.29 | 1.00 | 0.95 | 0.91 |
| g_ Peptostreptococcus | 0.0000 | 0.0001 | 0.0005 | 0.0002 | 0.0001 | 23.47 | 1.00 | 1.00 | 1.00 |
| g_Modestobacter | 0.0000 | 0.0002 | 0.0014 | 0.0007 | 0.0001 | 27.86 | 1.00 | 1.00 | 1.00 |
| g_ Tepidimonas | 0.0000 | 0.0001 | 0.0006 | 0.0004 | 0.0004 | 31.14 | 1.00 | 1.00 | 1.00 |
| g_ Dermacoccus | 0.0001 | 0.0002 | 0.0047 | 0.0005 | 0.0000 | 50.52 | 1.00 | 1.00 | 1.00 |
| g_ Janthinobacterium | 0.0000 | 0.0000 | 0.0006 | 0.0004 | 0.0013 | 65.90 | 1.00 | 1.00 | 1.00 |
| g_ Wautersiella | 0.0000 | 0.0001 | 0.0013 | 0.0010 | 0.0033 | 67.59 | 1.00 | 1.00 | 1.00 |
| g_Novosphingobium | 0.0001 | 0.0002 | 0.0050 | 0.0013 | 0.0000 | 71.05 | 1.00 | 1.00 | 1.00 |
| g_ Nesterenkonia | 0.0000 | 0.0001 | 0.0021 | 0.0010 | 0.0001 | 81.66 | 1.00 | 1.00 | 1.00 |
| g_Sneathia | 0.0001 | 0.0004 | 0.0115 | 0.0037 | 0.0000 | 98.04 | 1.00 | 1.00 | 1.00 |
| g_WAL_1855D | 0.0000 | 0.0000 | 0.0007 | 0.0004 | 0.0002 | 115.00 | 0.99 | 0.95 | 0.91 |
| g_Skermanella | 0.0000 | 0.0000 | 0.0007 | 0.0003 | 0.0001 | 194.32 | 1.00 | 1.00 | 1.00 |
| g_Shuttleworthia | 0.0000 | 0.0001 | 0.0079 | 0.0036 | 0.0000 | 232.06 | 1.00 | 1.00 | 1.00 |
| g_Roseateles | 0.0000 | 0.0000 | 0.0031 | 0.0018 | 0.0003 | 310.09 | 1.00 | 1.00 | 1.00 |
| g_ Bdellovibrio | 0.0000 | 0.0000 | 0.0019 | 0.0008 | 0.0000 | 871.83 | 1.00 | 1.00 | 1.00 |
| g_ Actinobaculum | 0.0000 | 0.0000 | 0.0013 | 0.0008 | 0.0000 | >100 | 1.00 | 1.00 | 1.00 |
| g_ Candidatus Solibacter | 0.0000 | 0.0000 | 0.0009 | 0.0011 | 0.0008 | >100 | 1.00 | 1.00 | 1.00 |
| g_Chroococcidiopsis | 0.0000 | 0.0000 | 0.0006 | 0.0004 | 0.0000 | >100 | 0.98 | 1.00 | 0.91 |

The above description of the present invention is provided only for illustrative purposes, and it will be understood by one of ordinary skill in the art to which the present invention pertains that the invention may be embodied in various modified forms without departing from the spirit or essential characteristics thereof. Thus, the embodiments described herein should be considered in an illustrative sense only and not for the purpose of limitation.

### [Industrial Applicability]

The method of providing information for diagnosing depression through a bacterial metagenomic analysis according to the present invention may be used for predicting the risk of depression onset and diagnosing depression by performing a bacterial metagenomic analysis using subject-derived samples to analyze an increase or decrease in the content of specific bacteria-derived extracellular vesicles.

## Claims

1. A method of providing information for depression diagnosis, comprising the following processes:
(a) extracting DNAs from extracellular vesicles isolated from subject samples;
(b) performing polymerase chain reaction (PCR) on the extracted DNA using a pair of primers comprising SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

2. The method of claim 1, wherein process (c) comprises comparing an increase or decrease in content of extracellular vesicles derived from one or more bacteria selected from the group consisting of the phylum *Verrucomicrobia*, the phylum *Bacteroidetes,* the phylum *Actinobacteria,* the phylum *Cyanobacteria,* the phylum *Thermi,* the phylum *Fusobacteria,* the phylum *Acidobacteria,* the phylum *Chloroflexi,* and the phylum *Armatimonadetes.*

3. The method of claim 1, wherein process (c) comprises comparing an increase or decrease in content of extracellular vesicles derived from one or more bacteria selected from the group consisting of the class *Erysipelotrichi,* the class *Verrucomicrobiae*, the class *Bacteroidia,* the class *Clostridia,* the class *Chloroplast,* the class *Actinobacteria,* the class *Alphaproteobacteria,* the class *Deltaproteobacteria,* the class *Saprospirae,* the class *Flavobacteriia,* the class *Cytophagia,* the class *Deinococci,* the class *Sphingobacteriia,* the class *Fusobacteriia*, the class *Fimbriimonadia,* the class TM7-1, the class *Pedosphaerae,* the class *Oscillatoriophycideae,* the class *Anaerolineae,* the class *Acidobacteria*-6, the class *Solibacteres,* the class *Nostocophycideae,* and the class iii1-8.

4. The method of claim 1, wherein process (c) comprises comparing an increase or decrease in content of extracellular vesicles derived from one or more bacteria selected from the group consisting of the order *Turicibacterales,* the order *Erysipelotrichales,* the order *Verrucomicrobiales*, the order RF39, the order *Bacteroidales,* the order *Enterobacteriales,* the order *Clostridiales,* the order *Pasteurellales*, the order *Sphingomonadales,* the order *Bacillales,* the order *Xanthomonadales,* the order *Streptophyta,* the order *Rhodobacterales,* the order *Caulobacterales,* the order *Actinomycetales,* the order *Saprospirales*, the order *Rhizobiales,* the order *Flavobacteriales,* the order *Cytophagales,* the order *Myxococcales,* the order *Rickettsiales,* the order *Thermales,* the order *Rhodospirillales,* the order *Deinococcales,* the order *Sphingobacteriales,* the order *Fusobacteriales,* the order *Fimbriimonadales*, the order BD7-3, the order *Pedosphaerales,* the order *Aeromonadales,* the order *Chroococcales,* the order 1025, the order *Bdellovibrionales,* the order iii1-15, the order *Solibacterales*, the order PYR10d3, and the order DS-18.

5. The method of claim 1, wherein process (c) comprises comparing an increase or decrease in content of extracellular vesicles derived from one or more bacteria selected from the group consisting of the family *Turicibacteraceae,* the family *Erysipelotrichaceae,* the family *Verrucomicrobiaceae,* the family *Rikenellaceae,* the family *Bacteroidaceae,* the family *Odoribacteraceae,* the family S24-7, the family *Clostridiaceae,* the family *Ruminococcaceae*, the family *Enterococcaceae,* the family *Paraprevotellaceae,* the family *Enterobacteriaceae,* the family *Leuconostocaceae,* the family *Porphyromonadaceae*, the family *Prevotellaceae,* the family *Pasteurellaceae,* the family *Actinomycetaceae,* the family *Burkholderiaceae*, the family *Xanthomonadaceae*, the family *Intrasporangiaceae,* the family *Staphylococcaceae,* the family *Sphingomonadaceae,* the family *Propionibacteriaceae,* the family *Nocardiaceae,* the family *Lactobacillaceae,* the family *Methylobacteriaceae,* the family *Rhodobacteraceae,* the family *Tissierellaceae*, the family *Acetobacteraceae,* the family *Caulobacteraceae,* the family *Nocardioidaceae*, the family *Hyphomicrobiaceae,* the family *Chitinophagaceae,* the family *Chromatiaceae,* the family *Flavobacteriaceae,* the family *Micrococcaceae,* the family *Sphingobacteriaceae,* the family *Corynebacteriaceae,* the family *Cytophagaceae,* the family *Weeksellaceae*, the family *Sinobacteraceae,* the family *Thermaceae,* the family *Deinococcaceae,* the family *Rhizobiaceae,* the family *Mycobacteriaceae,* the family *Comamonadaceae,* the family *Bacillaceae,* the family *Dermabacteraceae,* the family *Erythrobacteraceae,* the family *Geodermatophilaceae,* the family *Fimbriimonadaceae*, the family *Bartonellaceae,* the family *Leptotrichiaceae,* the family *Dermacoccaceae,* the family *Rhodospirillaceae,* the family *Aeromonadaceae*, the family Rs-045, the family *Xenococcaceae,* the family *Sporichthyaceae,* the family *Bdellovibrionaceae,* and the family *Solibacteraceae.*

6. The method of claim 1, wherein process (c) comprises comparing an increase or decrease in content of extracellular vesicles derived from one or more bacteria selected from the group consisting of the genus *Butyricimonas,* the genus *Turicibacter,* the genus SMB53, the genus *Eubacterium,* the genus *Akkermansia*, the genus *Proteus,* the genus *Ruminococcus*, the genus *Parabacteroides,* the genus *Roseburia,* the genus *Bacteroides,* the genus *Phascolarctobacterium,* the genus *Sporosarcina,* the genus *Weissella,* the genus *Faecalibacterium,* the genus *Collinsella,* the genus *Oscillospira,* the genus *Prevotella,* the genus *Sphingomonas,* the genus *Finegoldia*, the genus *Chryseobacterium,* the genus *Lautropia,* the genus *Methylobacterium,* the genus *Propionibacterium,* the genus *Rhodococcus,* the genus *Flavobacterium,* the genus *Staphylococcus,* the genus *Lactobacillus,* the genus *Porphyromonas,* the genus *Peptoniphilus,* the genus *Adlercreutzia,* the genus *Erwinia,* the genus *Citrobacter,* the genus *Microbispora,* the genus *Corynebacterium,* the genus *Leptotrichia,* the genus *Pedobacter,* the genus *Enhydrobacter,* the genus *Cloacibacterium,* the genus *Paracoccus,* the genus *Kaistobacter,* the genus *Thermus*, the genus *Actinobacillus,* the genus *Deinococcus,* the genus *Anaerococcus,* the genus *Hymenobacter,* the genus *Achromobacter,* the genus *Mycobacterium,* the genus *Agrobacterium,* the genus *Brachybacterium,* the genus *Capnocytophaga*, the genus *Bacillus,* the genus *Arthrobacter,* the genus *Megasphaera*, the genus *Fimbriimonas,* the genus *Peptostreptococcus,* the genus *Modestobacter,* the genus *Tepidimonas*, the genus *Dermacoccus*, the genus *Janthinobacterium*, the genus *Wautersiella*, the genus *Novosphingobium,* the genus *Nesterenkonia,* the genus *Sneathia,* the genus WAL_1855D, the genus *Skermanella,* the genus *Shuttleworthia,* the genus *Roseateles,* the genus *Bdellovibrio,* the genus *Actinobaculum,* the genus *Candidatus Solibacter,* and the genus *Chroococcidiopsis.*

7. The method of claim 1, wherein the subject sample is urine.

8. A method of diagnosing depression, comprising the following processes:
(a) extracting DNAs from extracellular vesicles isolated from subject samples;
(b) performing polymerase chain reaction (PCR) on the extracted DNA using a pair of primers comprising SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

9. The method of claim 8, wherein process (c) comprises comparing an increase or decrease in content of extracellular vesicles derived from one or more bacteria selected from the group consisting of the phylum *Verrucomicrobia*, the phylum *Bacteroidetes,* the phylum *Actinobacteria,* the phylum *Cyanobacteria,* the phylum *Thermi,* the phylum *Fusobacteria,* the phylum *Acidobacteria,* the phylum *Chloroflexi,* and the phylum *Armatimonadetes.*

10. The method of claim 8, wherein process (c) comprises comparing an increase or decrease in content of extracellular vesicles derived from one or more bacteria selected from the group consisting of the class *Erysipelotrichi,* the class *Verrucomicrobiae*, the class *Bacteroidia,* the class *Clostridia,* the class *Chloroplast,* the class *Actinobacteria,* the class *Alphaproteobacteria,* the class *Deltaproteobacteria,* the class *Saprospirae,* the class *Flavobacteriia,* the class *Cytophagia,* the class *Deinococci,* the class *Sphingobacteriia,* the class *Fusobacteriia*, the class *Fimbriimonadia,* the class TM7-1, the class *Pedosphaerae,* the class *Oscillatoriophycideae,* the class *Anaerolineae,* the class *Acidobacteria*-6, the class *Solibacteres,* the class *Nostocophycideae,* and the class iii1-8.

11. The method of claim 8, wherein process (c) comprises comparing an increase or decrease in content of extracellular vesicles derived from one or more bacteria selected from the group consisting of the order *Turicibacterales,* the order *Erysipelotrichales,* the order *Verrucomicrobiales*, the order RF39, the order *Bacteroidales,* the order *Enterobacteriales,* the order *Clostridiales,* the order *Pasteurellales*, the order *Sphingomonadales,* the order *Bacillales,* the order *Xanthomonadales,* the order *Streptophyta,* the order *Rhodobacterales,* the order *Caulobacterales,* the order *Actinomycetales,* the order *Saprospirales*, the order *Rhizobiales,* the order *Flavobacteriales,* the order *Cytophagales,* the order *Myxococcales,* the order *Rickettsiales,* the order *Thermales,* the order *Rhodospirillales,* the order *Deinococcales,* the order *Sphingobacteriales,* the order *Fusobacteriales,* the order *Fimbriimonadales*, the order BD7-3, the order *Pedosphaerales,* the order *Aeromonadales,* the order *Chroococcales,* the order 1025, the order *Bdellovibrionales,* the order iii1-15, the order *Solibacterales,* the order PYR10d3, and the order DS-18.

12. The method of claim 8, wherein process (c) comprises comparing an increase or decrease in content of extracellular vesicles derived from one or more bacteria selected from the group consisting of the family *Turicibacteraceae,* the family *Erysipelotrichaceae,* the family *Verrucomicrobiaceae,* the family *Rikenellaceae,* the family *Bacteroidaceae,* the family *Odoribacteraceae,* the family S24-7, the family *Clostridiaceae,* the family *Ruminococcaceae*, the family *Enterococcaceae,* the family *Paraprevotellaceae,* the family *Enterobacteriaceae,* the family *Leuconostocaceae,* the family *Porphyromonadaceae*, the family *Prevotellaceae,* the family *Pasteurellaceae,* the family *Actinomycetaceae,* the family *Burkholderiaceae*, the family *Xanthomonadaceae*, the family *Intrasporangiaceae,* the family *Staphylococcaceae,* the family *Sphingomonadaceae,* the family *Propionibacteriaceae,* the family *Nocardiaceae,* the family *Lactobacillaceae,* the family *Methylobacteriaceae,* the family *Rhodobacteraceae,* the family *Tissierellaceae*, the family *Acetobacteraceae,* the family *Caulobacteraceae,* the family *Nocardioidaceae*, the family *Hyphomicrobiaceae,* the family *Chitinophagaceae,* the family *Chromatiaceae,* the family *Flavobacteriaceae,* the family *Micrococcaceae,* the family *Sphingobacteriaceae,* the family *Corynebacteriaceae,* the family *Cytophagaceae,* the family *Weeksellaceae*, the family *Sinobacteraceae,* the family *Thermaceae,* the family *Deinococcaceae,* the family *Rhizobiaceae,* the family *Mycobacteriaceae,* the family *Comamonadaceae,* the family *Bacillaceae,* the family *Dermabacteraceae,* the family *Erythrobacteraceae,* the family *Geodermatophilaceae,* the family *Fimbriimonadaceae*, the family *Bartonellaceae,* the family *Leptotrichiaceae,* the family *Dermacoccaceae,* the family *Rhodospirillaceae,* the family *Aeromonadaceae*, the family Rs-045, the family *Xenococcaceae,* the family *Sporichthyaceae,* the family *Bdellovibrionaceae,* and the family *Solibacteraceae.*

13. The method of claim 8, wherein process (c) comprises comparing an increase or decrease in content of extracellular vesicles derived from one or more bacteria selected from the group consisting of the genus *Butyricimonas,* the genus *Turicibacter,* the genus SMB53, the genus *Eubacterium,* the genus *Akkermansia*, the genus *Proteus,* the genus *Ruminococcus*, the genus *Parabacteroides,* the genus *Roseburia,* the genus *Bacteroides,* the genus *Phascolarctobacterium,* the genus *Sporosarcina,* the genus *Weissella,* the genus *Faecalibacterium,* the genus *Collinsella,* the genus *Oscillospira,* the genus *Prevotella,* the genus *Sphingomonas,* the genus *Finegoldia*, the genus *Chryseobacterium,* the genus *Lautropia,* the genus *Methylobacterium,* the genus *Propionibacterium,* the genus *Rhodococcus,* the genus *Flavobacterium,* the genus *Staphylococcus,* the genus *Lactobacillus,* the genus *Porphyromonas,* the genus *Peptoniphilus,* the genus *Adlercreutzia,* the genus *Erwinia,* the genus *Citrobacter,* the genus *Microbispora,* the genus *Corynebacterium,* the genus *Leptotrichia,* the genus *Pedobacter,* the genus *Enhydrobacter*, the genus *Cloacibacterium,* the genus *Paracoccus,* the genus *Kaistobacter,* the genus *Thermus*, the genus *Actinobacillus,* the genus *Deinococcus,* the genus *Anaerococcus,* the genus *Hymenobacter,* the genus *Achromobacter,* the genus *Mycobacterium,* the genus *Agrobacterium,* the genus *Brachybacterium*, the genus *Capnocytophaga*, the genus *Bacillus,* the genus *Arthrobacter,* the genus *Megasphaera*, the genus *Fimbriimonas,* the genus *Peptostreptococcus,* the genus *Modestobacter,* the genus *Tepidimonas*, the genus *Dermacoccus,* the genus *Janthinobacterium,* the genus *Wautersiella*, the genus *Novosphingobium,* the genus *Nesterenkonia,* the genus *Sneathia*, the genus WAL_1855D, the genus *Skermanella,* the genus *Shuttleworthia,* the genus *Roseateles,* the genus *Bdellovibrio*, the genus *Actinobaculum,* the genus *Candidatus Solibacter,* and the genus *Chroococcidiopsis.*

14. The method of claim 8, wherein the subject sample is urine.
